# EUROPEAN PATENT SPECIFICATION

(11) **EP 3 648 686 B1**
(45) Date of publication and mention of the grant of the patent: **15.09.2021**
(21) Application number: 18745794.0
(22) Date of filing: 05.07.2018
(51) Int. Cl.: A61B 17/221

(54) **INVERTING THROMBECTOMY APPARATUSES**
VORRICHTUNGEN FÜR INVERTIERENDE THROMBEKTOMIE
APPAREILS DE THROMBECTOMIE PAR INVERSION

(30) Priority: 06.07.2017 US 201762529386 P
(43) Date of publication of application: 13.05.2020
(73) Proprietor: Stryker Corporation, Fremont, California 94538 (US)
(72) Inventor: WALLACE, Michael, P., Pleasanton, California 94566 (US); GARABEDIAN, Robert, J., Mountain View, California 94040 (US); WALLACE, Gavin, P., Pleasanton, California 94566 (US)
(74) Representative: Lecomte & Partners
(86) International application number: PCT/US2018/040937
(87) International publication number: WO 2019/010318

(56) References cited:
- AU-A1- 2015 210 338
- US-A- 5 971 938
- US-A1- 2005 177 132
- US-A1- 2010 249 815
- US-A1- 2013 226 196
- US-A1- 2015 196 380
- US-A1- 2016 074 627
- US-A1- 2017 086 864

## Description

### FIELD

The apparatuses and exemplary methods disclosed and described herein relate to mechanical removal of objects from within a body lumen, such as a blood vessel. In particular, disclosed and described herein are mechanical thrombectomy apparatuses and exemplary methods of using same.

### BACKGROUND

Many vascular problems stem from insufficient blood flow through blood vessels. One causes of insufficient or irregular blood flow is a blockage within a blood vessel referred to as a blood clot, or thrombus. Thrombi can occur for many reasons, including after a trauma such as surgery, or due to other causes. For example, a large percentage of the more than 1.2 million heart attacks in the United States are caused by blood clots (thrombi) which form within a coronary artery. It is often desirable to remove tissue from the body in a minimally invasive manner as possible, so as not to damage other tissues. For example, removal of tissue, such as blood clots, from within a patient's vasculature may improve patient conditions and quality of life.

When a thrombus forms, it may effectively stop the flow of blood through the zone of formation. If the thrombus extends across the interior diameter of an artery, it may cut off the flow of blood through the artery. If one of the coronary arteries is 100% thrombosed, the flow of blood is stopped in that artery, resulting in a shortage of oxygen carrying red blood cells, e.g., to supply the muscle (myocardium) of the heart wall. Such a thrombosis is unnecessary to prevent loss of blood but can be undesirably triggered within an artery by damage to the arterial wall from atherosclerotic disease. Thus, the underlying disease of atherosclerosis may not cause acute oxygen deficiency (ischemia) but can trigger acute ischemia via induced thrombosis. Similarly, thrombosis of one of the carotid arteries can lead to stroke because of insufficient oxygen supply to vital nerve centers in the cranium. Oxygen deficiency reduces or prohibits muscular activity, can cause chest pain (angina pectoris), and can lead to death of myocardium which permanently disables the heart to some extent. If the myocardial cell death is extensive, the heart will be unable to pump sufficient blood to supply the body's life sustaining needs. The extent of ischemia is affected by many factors, including the existence of collateral blood vessels and flow which can provide the necessary oxygen.

Clinical data indicates that clot removal may be beneficial or even necessary to improve outcomes. For example, in the peripheral vasculature, interventions and procedures can reduce the need for an amputation by as much as 80 percent. The ultimate goal of any modality to treat these conditions of the arterial or venous system is to remove the blockage or restore patency, quickly, safely, and cost effectively. This may be achieved by thrombus dissolution, fragmentation, thrombus aspiration or a combination of these methods.

Depending on the size, location and extent of a clot, it may be particularly advantageous to employ a mechanical thrombectomy device to remove the clot in a manner that is both safe and effective.

US 2017/0086864 A1 discloses a mechanical thrombectomy apparatus according to the preamble of claim 1. Operations of advancing the thrombectomy apparatus into a tortuous anatomy may under certain circumstances show difficulties.

Described herein are mechanical thrombectomy apparatuses (devices, systems and kit) and exemplary methods of using and making such apparatuses that may address the needs and problems discussed above.

### SUMMARY OF THE DISCLOSURE

The invention is directed to a mechanical thrombectomy apparatuse according to claim 1. In particular, described herein are mechanical thrombectomy apparatuses having improved rolling/inverting which may be particularly well adapted for removing large and hard clots, and/or difficult to grasp clots, from within a vessel.

Further developments of the invention are according to dependent claims 2-14.

### BRIEF DESCRIPTION OF THE DRAWINGS

The various features and aspects of the inventions disclosed and described herein are set forth with particularity in the claims that follow. A better understanding of the features and advantages of the disclosed and described apparatuses and methods will be obtained by reference to the following detailed description when read with the accompanying drawings, of which:

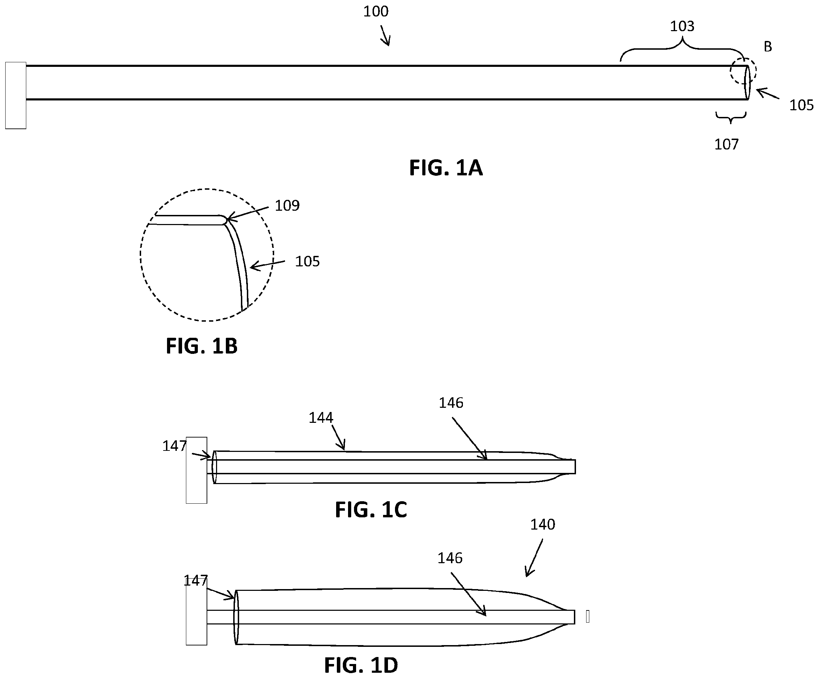

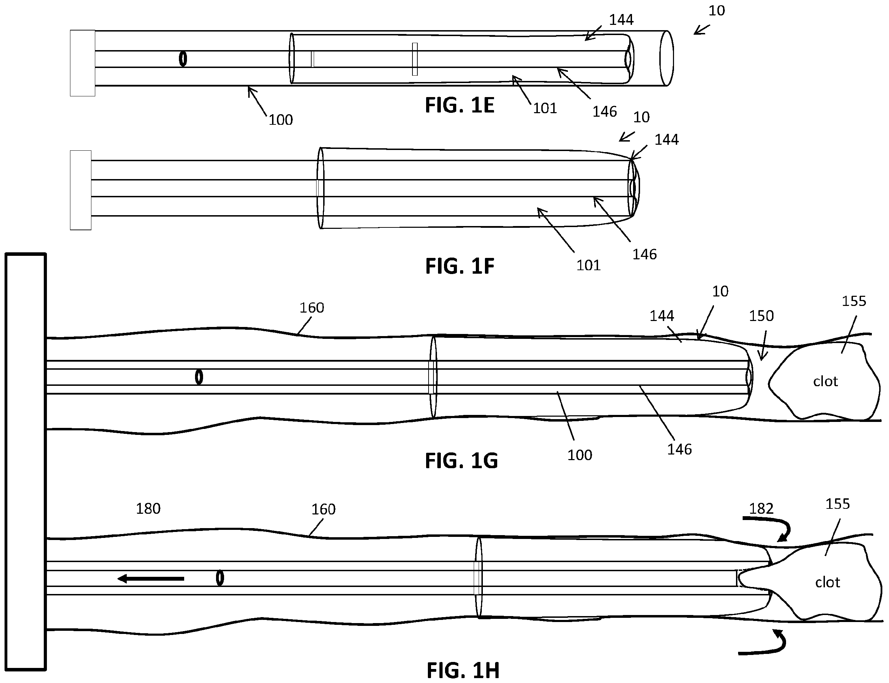

FIGS. 1A-1H illustrate an example of an apparatus for mechanically removing an object such as a clot form a body region. FIG. 1A shows an example of an elongate inversion support catheter portion of an apparatus. At least the distal end of the elongate inversion support is configured as a catheter. FIG. 1B shows an enlarged view of a partial section of a distal end (opening) of the catheter of the elongate inversion support of FIG. 1A, showing the aperture formed by the distal end opening; FIG. 1C shows an example of a flexible tractor tube attached over a puller (the puller in this example is configured as a catheter). The tractor is shown in a first configuration. In some variations, the flexible tractor tube may be biased open, e.g., by heat setting, to have an outer diameter that is greater than the inner diameter of the catheter of the elongate inversion support, as shown in FIG. ID. FIG. ID shows the same distal tractor region of FIG. 1C with the expandable first end region expanded. This first configuration may be compressed down into the elongate inversion support and the distal end inverted over the catheter portion of the elongate inversion support, as shown in FIG. 1E. In FIG. IE, the assembled mechanical thrombectomy apparatus includes an elongate inversion support catheter having a distal end and a distal end opening; the elongate puller extends within the elongate inversion support catheter and a flexible tractor tube is connected at one end to the elongate puller. The flexible tractor tube and puller may be extended through the elongate inversion support catheter, including extended out of the distal end (e.g., by pulling the elongate inversion support catheter proximally and/or by pushing the elongate puller distally). The flexible tractor tube may initially be held in a collapsed first configuration (as shown in FIG. IE) for positioning within a vessel; it may be deployed and expanded, as shown in FIG. IF, with the elongate inversion support catheter positioned between the flexible tractor tube and the elongate puller. The flexible tractor tube may be biased so that in the second configuration (inverted over the distal end of the catheter), the tractor has a 'relaxed' outer diameter that is greater than the outer diameter of the catheter of the elongate inversion support.
FIGS. 1G and 1H illustrate the use of an apparatus such as the one shown in FIGS. 1E and 1F to remove a clot by drawing the flexible tractor tube proximally and/or advancing the catheter distally towards the clot so that the expandable first end region inverts as it is drawn into the distal end of the catheter, pulling the clot into the catheter.

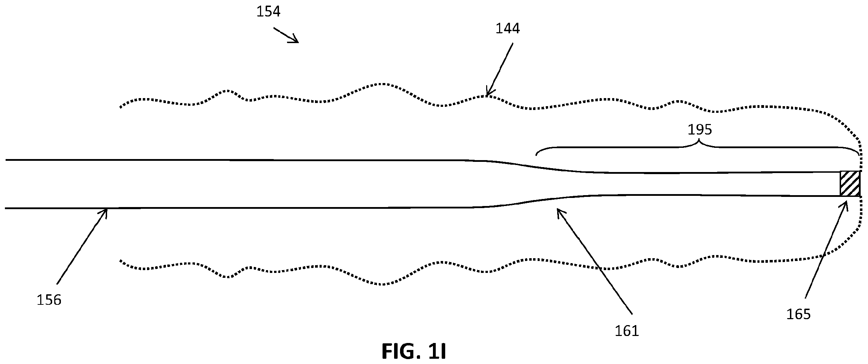

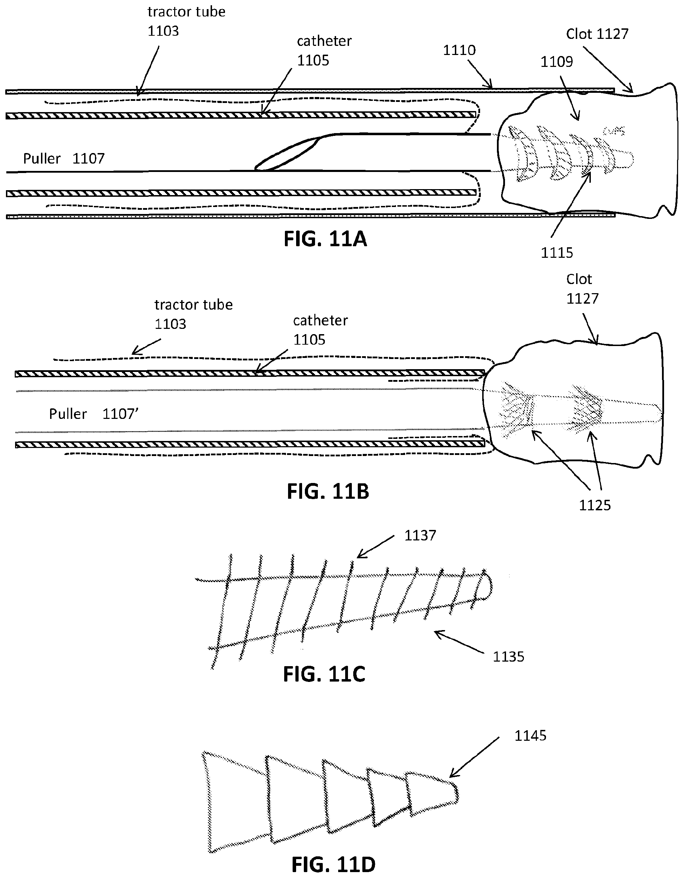

FIG. 1I illustrates an alternative variation of a tractor and puller. In FIG. 11, the tractor is shown attached to the distal end of a tapered or narrow puller; the distal end region is tapered, and includes a radiopaque marker at or near the attachment site to the tractor; the tractor may be knitted, braided, woven, etc. Thus, in some variations the distal end region of the puller may have a greater flexibility than the proximal end of the puller. The puller may be hollow (e.g., a catheter or hypotube) or solid (e.g., like a wire).

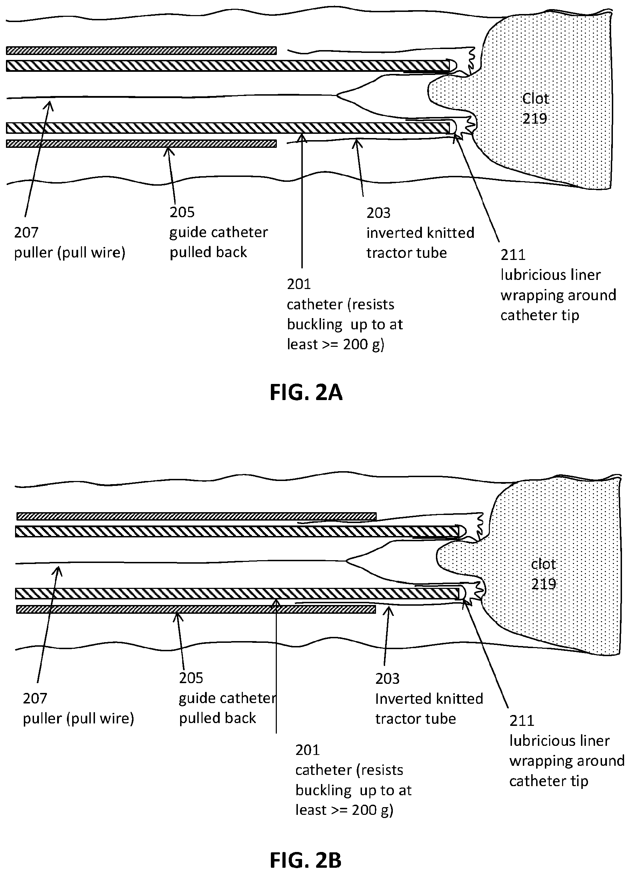

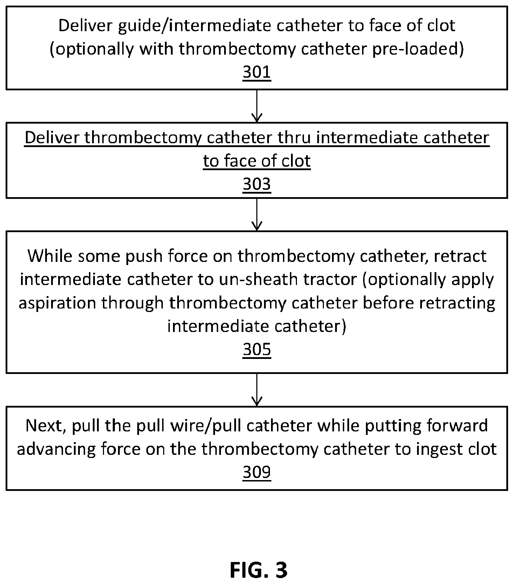

FIG. 2A illustrates a first mechanical thrombectomy apparatus and exemplary method of using it to remove a clot. In FIG. 2A, the apparatus includes a lubricious liner that is inverted over the distal end opening of the elongate inversion support catheter. FIG. 3 is a flowchart illustrating the method shown in FIG. 2A.
FIG. 2B is the mechanical thrombectomy apparatus of FIG. 2A, and an alternative exemplary method of using it to remove a clot. In this example, the outer catheter ("intermediate catheter") is only partially withdrawn so that the distal end of the tractor tube (shown as a knit tractor tube) is still held in place against the outer wall of the elongate inversion support catheter portion by the intermediate catheter.
FIG. 3 is a schematic illustrating an exemplary method of operating a mechanical thrombectomy apparatus such as the one shown in FIG. 2A.

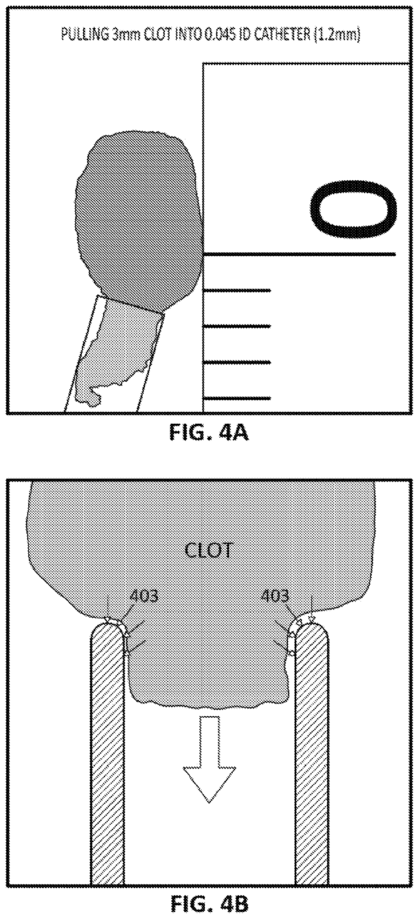

FIG. 4A is an example of a large (e.g., 3mm) and hard clot into a smaller ID catheter (e.g., 0.045 ID catheter of 1.2 mm opening diameter). FIG. 4B illustrates the normal forces on the inner and distal ends of the elongate inversion support catheter wall. The use of a low-friction material, such as a Teflon sleeve, extending around the distal end opening of the elongate inversion support catheter portion may prevent jamming and aid in compacting the hard clot.

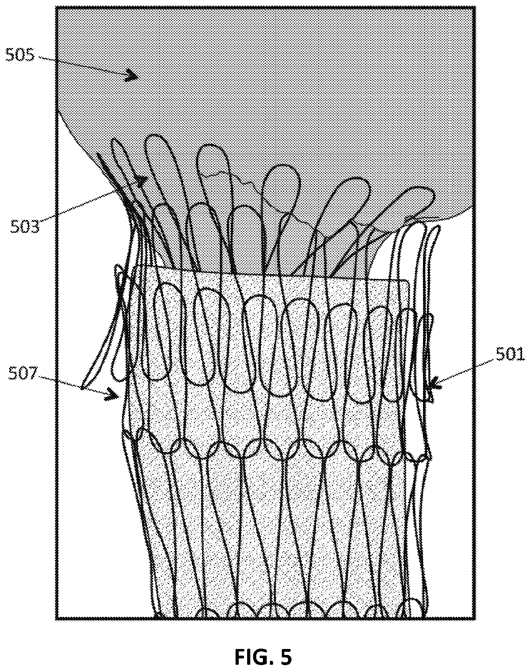

FIG. 5 illustrates a knit tractor tube that may also aid in pulling a large-diameter clot into an elongate inversion support catheter. In FIG. 5, the loops forming the knit are interlocked, but may swing outward as the knit is rolled over the distal end opening of the elongate inversion support catheter, which may knead and/or masticate the clot, aiding in pulling it into the elongate inversion support catheter.

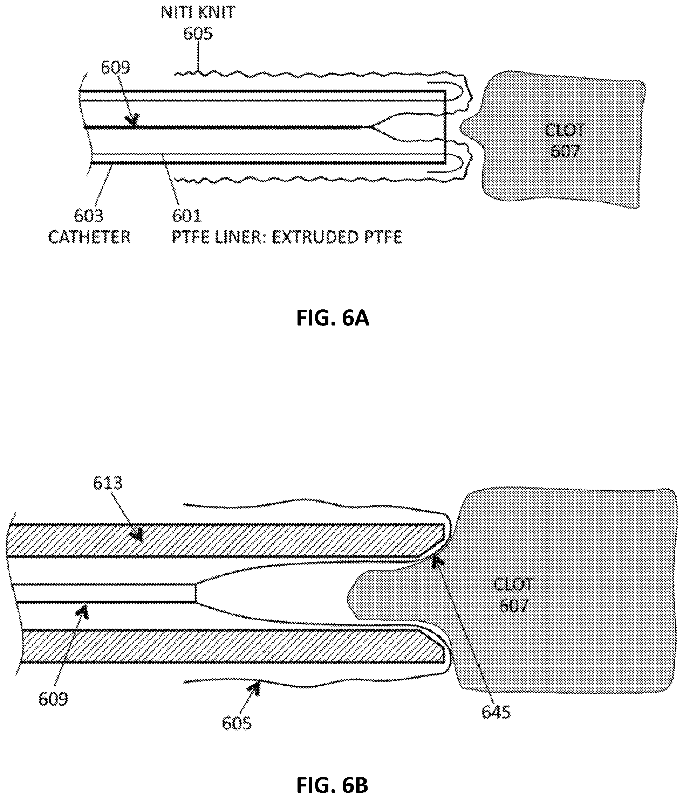

FIG. 6A illustrates an elongate inversion support catheter of a mechanical atherectomy apparatus including a low-friction liner (e.g., a PTFE liner) extended around the catheter tip to reduce pull friction.
FIG. 6B illustrates a modified tip for an elongate inversion support catheter, having a chamfer on the inner radius of the catheter to reduce pulling forces.

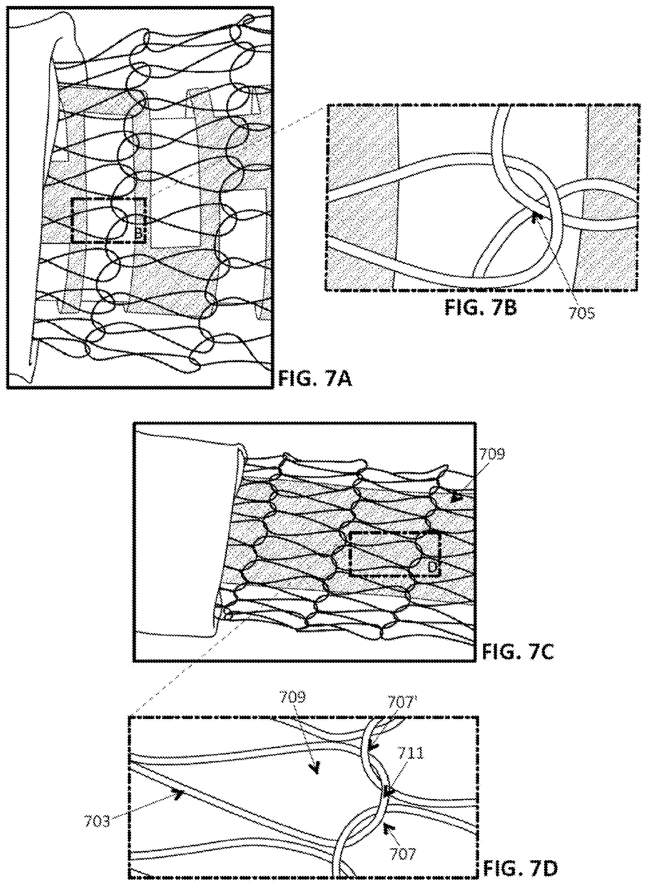

FIGS. 7A and 7B show an example of a knit tractor tube that undesirably includes regions where adjacent links of the knit include triple crossover points, e.g., as shown in greater detail in FIG 7B. Typically, when the diameter of the knitted tractor tube is greater than the outer diameter of the elongate inversion support catheter such that the adjacent links form triple cross-over points, as shown in FIG. 7B, the resulting tractor tube may roll move less smoothly (and/or may jam) over the distal end opening of the elongate inversion support catheter. In contrast, FIG. 7C and 7C shows an example of a knitted tractor tube having only double overlaps, which may more desirably roll over the elongate inversion support catheter. In FIG. 7D, an enlarged view of the links of the woven tractor tube is shown.

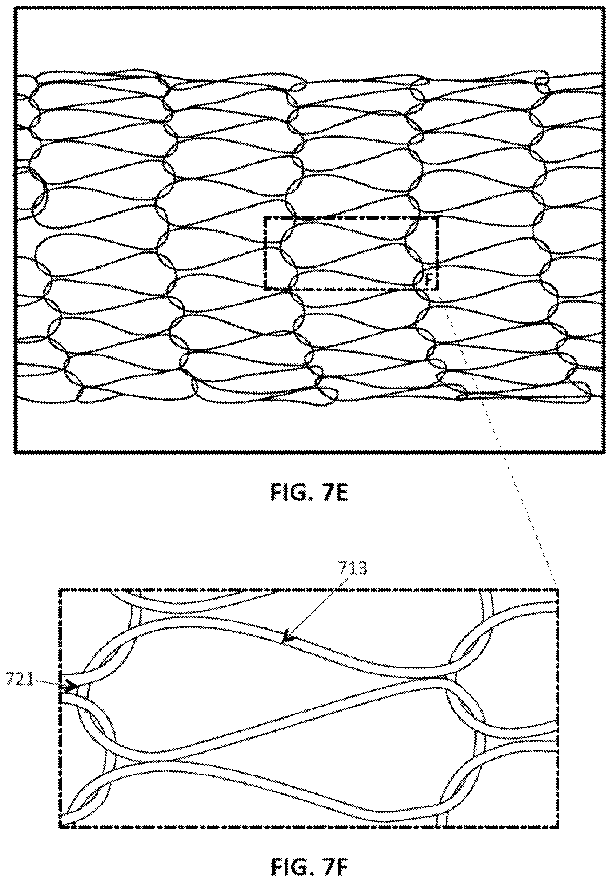

FIG. 7E and 7F illustrates a non-inverted knitted tube that is less desirable, as it may jam or prevent smooth rolling, despite having links that are the same dimension (and formed of the same materials) as the inverted knit shown in FIG. 7C and 7D. FIG 7F is an enlarged view of FIG. 7E.

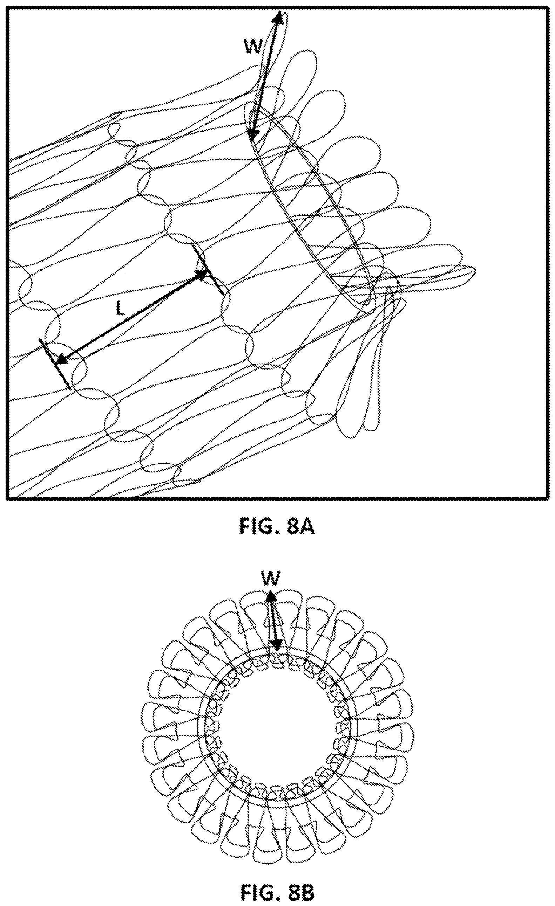

FIG. 8A is a side view of a distal end of a elongate inversion support catheter with a knitted tractor tube rolling into the distal end opening, illustrating tuning of the length, L, of the woven links to achieve a desired grab width, W, e.g., between 30-90% of L, for grabbing a clot with a woven tractor as described herein. FIG. 8B is an end-on view of the apparatus of FIG. 8A.

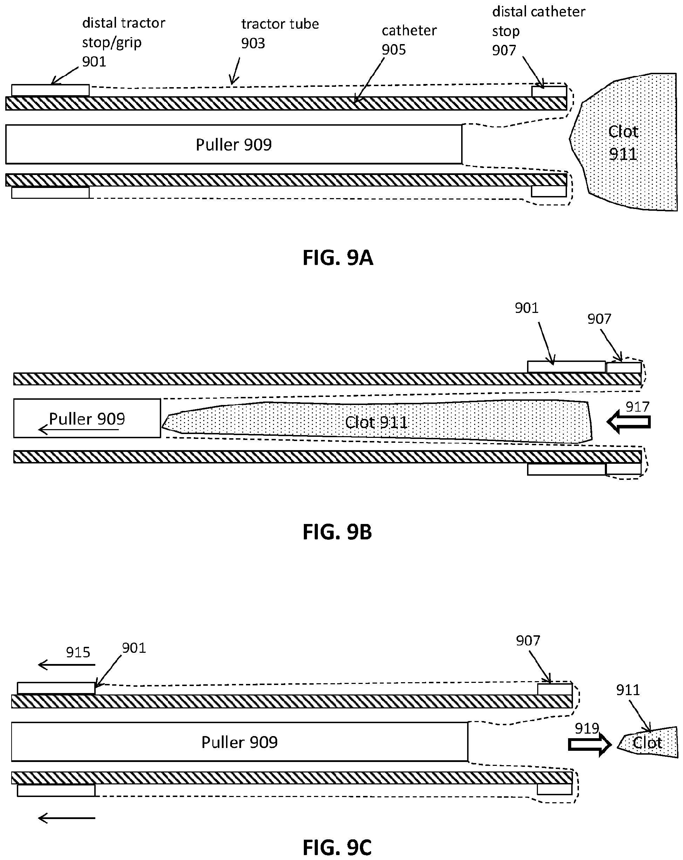

FIGS. 9A-9C illustrate an example of a mechanical thrombectomy apparatus that is configured as a reusable apparatus. In FIG. 9A, the apparatus includes a distal tractor stop/grip portion that prevents the tractor tube from inverting fully into the elongate inversion support catheter. Once the clot is pulled into the elongate inversion support catheter as shown in FIG. 9B, the apparatus may be removed from the body, and the clot ejected, e.g., by sliding the tractor tube along the outer diameter of the elongate inversion support catheter, as shown in FIG. 9C. The apparatus may then be re-used.

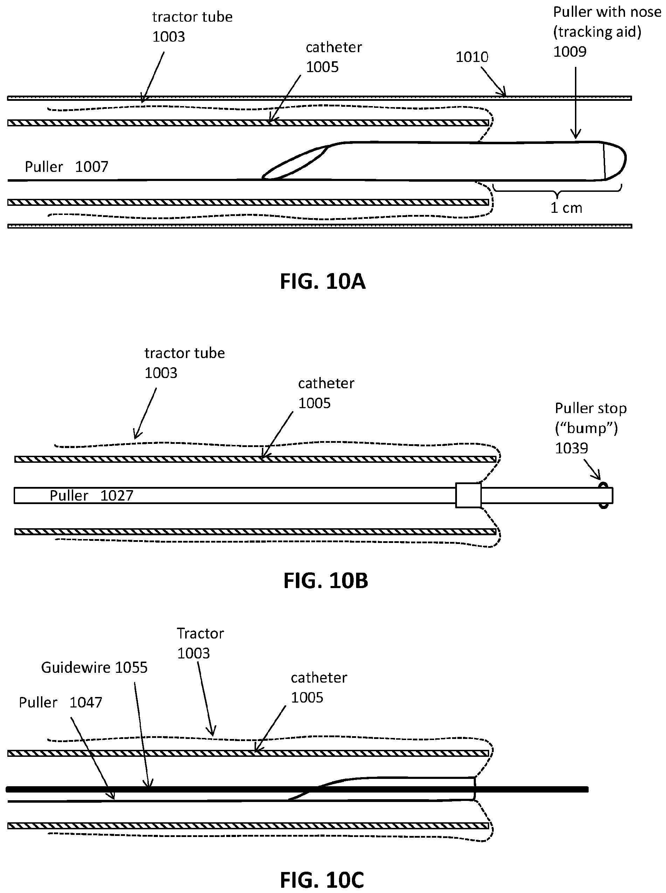

FIGS. 10A-10C illustrate variations of mechanical thrombectomy apparatuses according to the invention, that may have improved tracking, e.g., within a vessel (e.g., lumen) and/or within the lumen of a catheter, such as an intermediate catheter. FIG. 10A show an example in which the elongate puller is adapted to include a nose region at the distal end, including a bumper. The distal nose region/bumper may extend out from the elongate inversion support catheter until the target location, near the clot, has been reached, and then retracted proximally to roll the tractor tube into the elongate inversion support catheter. FIG. 10B is another example in which the puller (e.g. pull wire,in accordance with the invention or pull catheter) is slidable within a collar coupled to the tractor tube, but further includes an engagement region at or near the distal end of the puller that engages the collar and allows the puller to pull the tractor tube proximally in the elongate inversion support catheter to roll the tractor tube into the elongate inversion support catheter. FIG. 10C is another example in which the puller is a pull wire having a collar region near the distal end through which a guidewire may pass.
FIGS. 11A-11B illustrate another example of a mechanical thrombectomy according to the invention, that may have improved tracking, pulling of a clot and/or inverting of the tractor tube over the elongate inversion support catheter. FIG. 11A shows a puller (which may be solid or cannulated) having a distal tip region that includes a plurality of flanges or edges extending therefrom. In FIG. 11A the distal tip includes a plurality of cup-like members extending from therefrom. FIG. 11B shows another example of a puller in which the distal end includes a plurality of projections extending proximally. In FIG. 11B, the projections may be fringe or fibers extending from the outer surface of the distal end of the puller, which may be tapered or cylindrical.
FIG. 11C shows an example of a distal tip of a puller configured to include a helical protrusion spiraling around the distal end.
FIG. 11D is an example of a distal end of a puller configured to have a stepped profile. In FIG. 11D, the stepped profile includes a plurality of (e.g., 5) different sized cones connected and arranged from larger (proximal) to smaller (distal). The cones may be separate or solid and fused together.

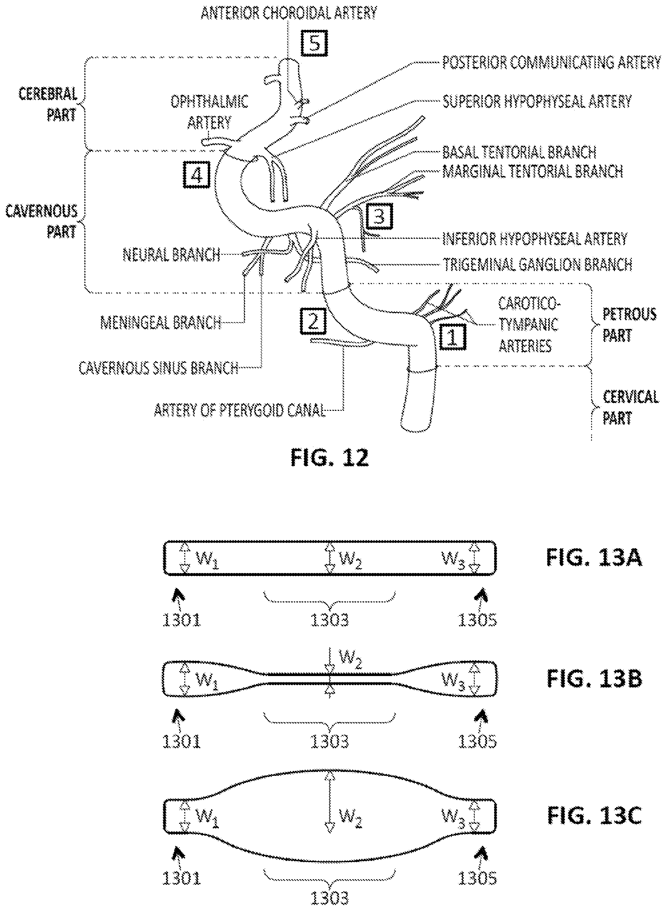

FIG. 12 is an example of an internal carotid artery, which has a tortious vascular path. This path may be divided up in to sub-regions, and a model of this path may be used to examine tracking of any of the apparatuses disclosed and described herein.
FIGS. 13A to 13C illustrate exemplary rectangular slots hat maybe formed in any of the inversion support catheters described herein. FIG. 13A shows slot that is in a neutral configuration, prior to heat setting in a collapsed or expanded configuration. In FIG. 13A, the slot has a width at the ends (w₁) that is approximately the same as the width in a middle region between the ends (w₂). In FIG. 13B, the slot has been compressed (e.g., by applying a longitudinal compression force on the catheter into which the slot is cut and heat setting in the compressed state) so that the width of the middle region (w₂) is less than the width of the end regions, which are not compressible (w₁). FIG. 13C shows a first open configuration, in which the slot has been pulled (e.g., by pulling a catheter in which the slot is formed in the longitudinal direction) to formed an open configuration, in which the middle region has a diameter (w₂) that is greater than the diameter of the end regions (w₁).

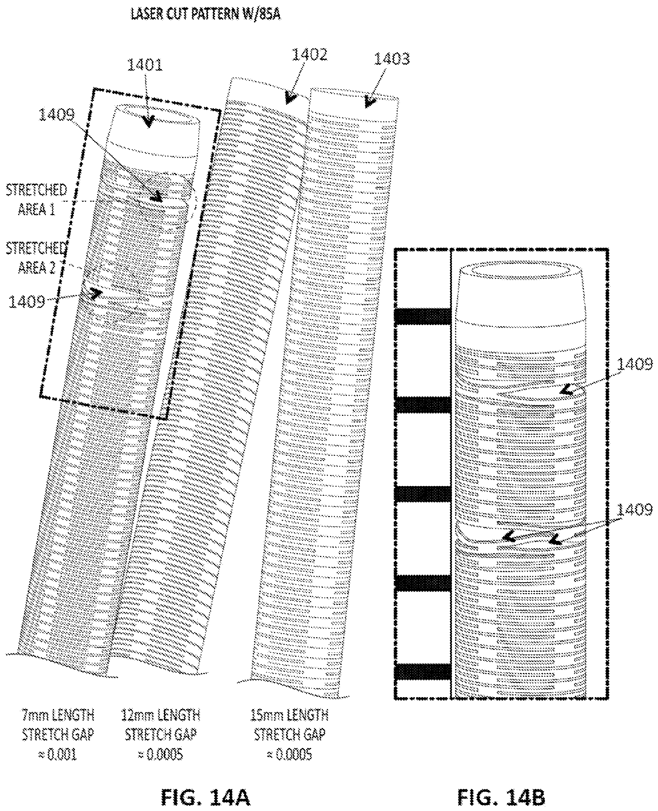

FIG. 14A shows three examples of inversion support catheters having a slotted pattern of cuts. FIG. 14B is an enlarged view of one of the inversion support catheters of FIG. 14A.

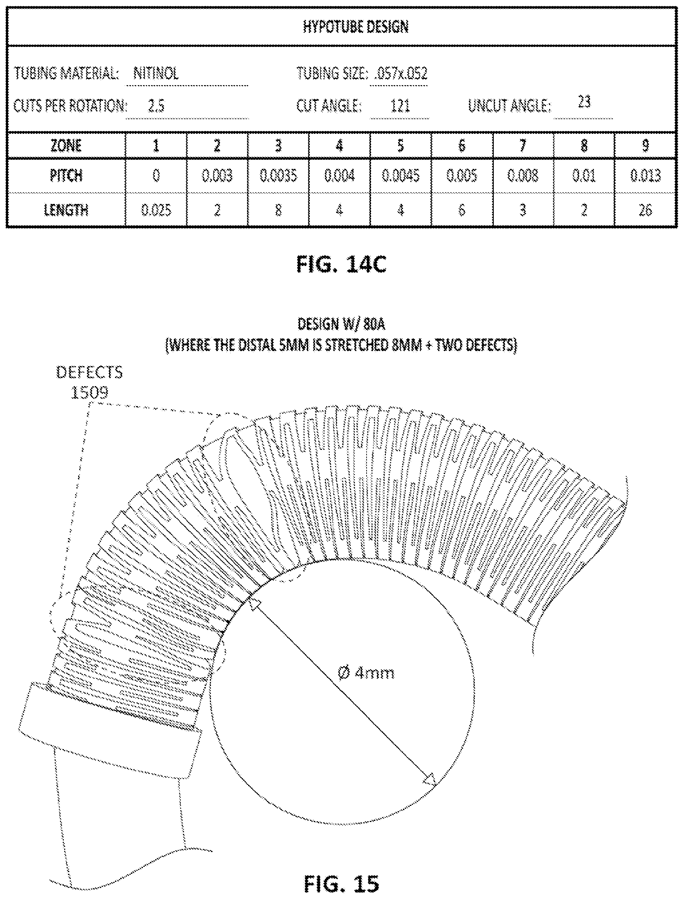

FIG. 14C describes a pattern used to form slotted inversion support catheter such as the ones shown in FIG. 14A.
FIG. 15 illustrates bending of a slotted inversion support catheter having a distal end region with a combination of open-cell and closed-cell slots cut cut into the distal ends; in this example, only two open-cell regions are included in the distal end region.

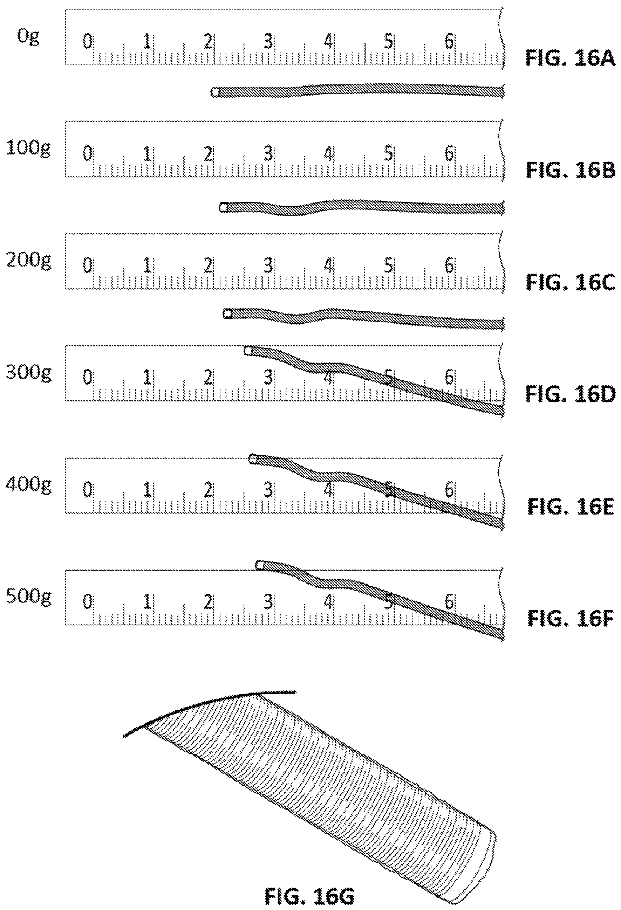

FIGS. 16A-16F illustrate increasing compression force applied to an exemplary slotted catheter, showing minimal compression/kinking. FIG. 16G shows the distal end of the device shown in FIGS. 16A-16F.

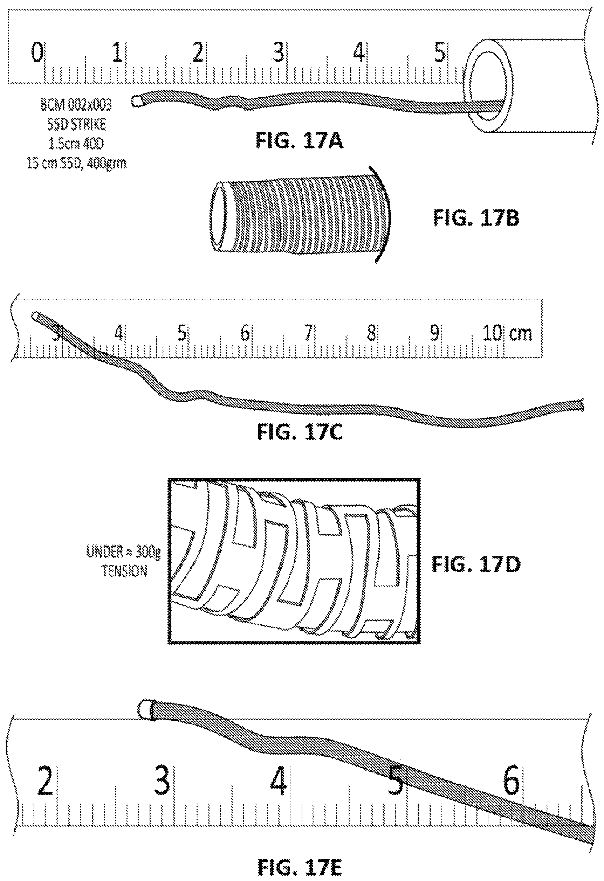

FIGS. 17A-17B illustrate another example of a slotted inversion support catheter undergoing 400 g of compression, showing kinking and shortening of the catheter. FIGS. 17C-17D illustrate a second example of a slotted inversion support catheter undergoing 400 g (FIG. 17C) and 300g (FIG. 17D) of compression; this example also kinked/shortened. FIG. 17D, shown for comparison, is the slotted inversion support catheter shown and described in FIG. 16A-16F, which did not substantially kink or shorten under 400g of compression. FIG. 17E illustrates another example of a slotted inversion support catheter similar to that shown in FIG. 16G, above.

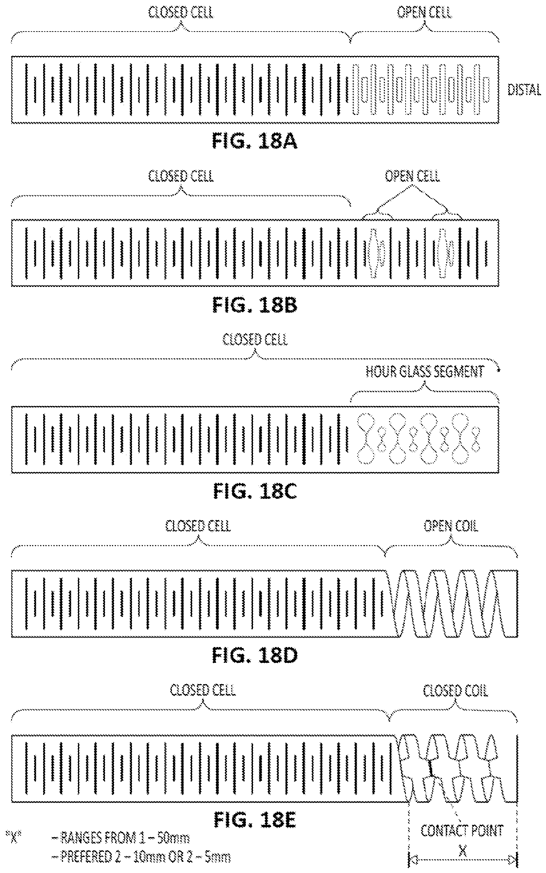

FIGS. 18A-18E illustrates examples of slotted inversion support catheters having proximal regions with close-cell slots and distal regions with open-cell cut out regions (including slots).

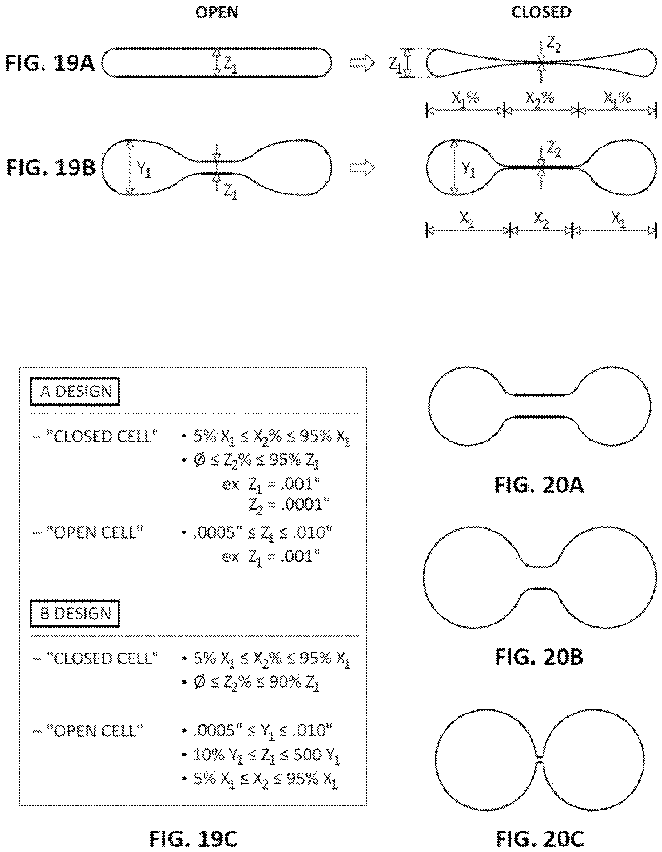

FIG. 19A illustrates an example of an approximately rectangular slot in an open-cell (left) and closed-cell (right) configuration. FIG. 19B illustrates an example of a lobed slot (e.g., centrally tapered, hourglass-shaped, barbell-shaped) slot in an open-cell and closed-cell configuration. In FIG. 19B, even the closed-cell configuration remains open at the ends, as shown. FIG. 19C provides exemplary dimensions of the slots shown in FIGS. 19A and 19B.
FIGS. 20A-20C illustrate examples of bi-lobed slots (e.g., centrally tapered, hourglass-shaped, barbell-shaped) similar to that shown in FIG. 19B.

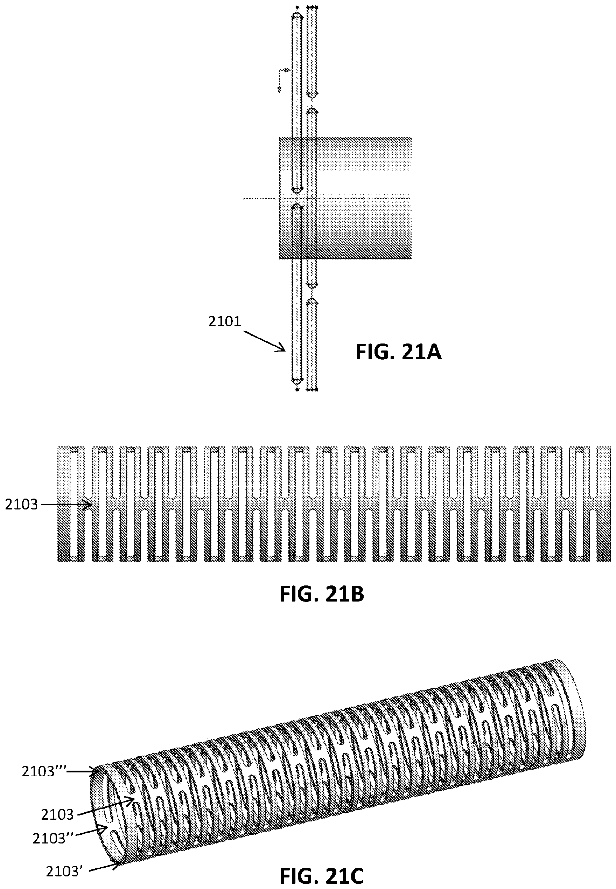

FIGS. 21A-21C illustrate another example of a slotted catheter having a brick-like pattern of aligned slots. FIG. 21A shows the repeating cut-out pattern that may be applied to a catheter (e.g., nitinol catheter tube). FIG 21B shows a side view of the slotted catheter. FIG. 21C shows a perspective view of the slotted catheter.

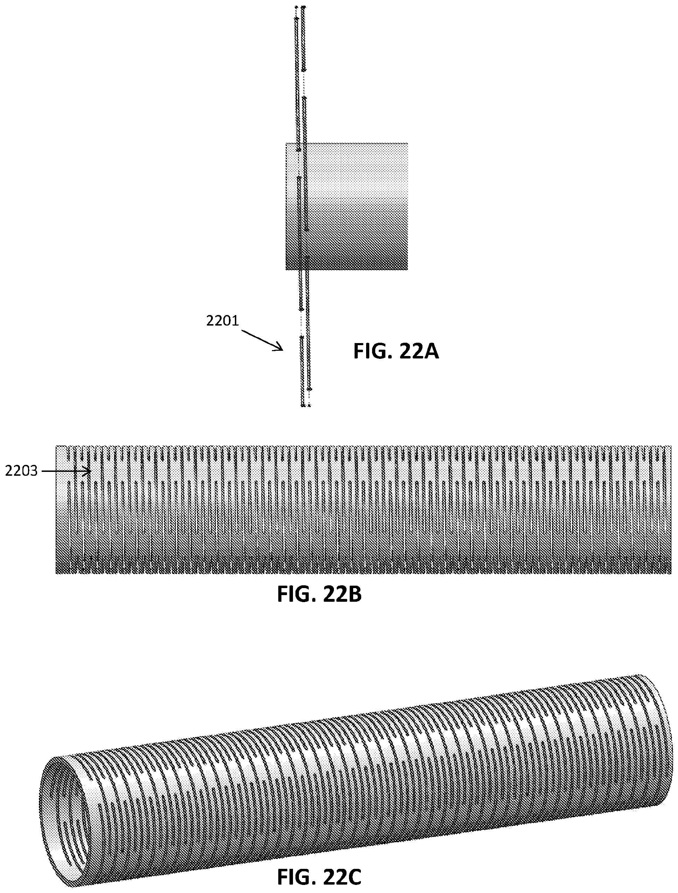

FIGS. 22A-22C illustrate another example of a slotted catheter having a spiral-like pattern of aligned slots. FIG. 22A shows the repeating cut-out pattern that may be applied to a catheter (e.g., nitinol catheter tube). FIG 22B shows a side view of the slotted catheter. FIG. 22C shows a perspective view of the slotted catheter.

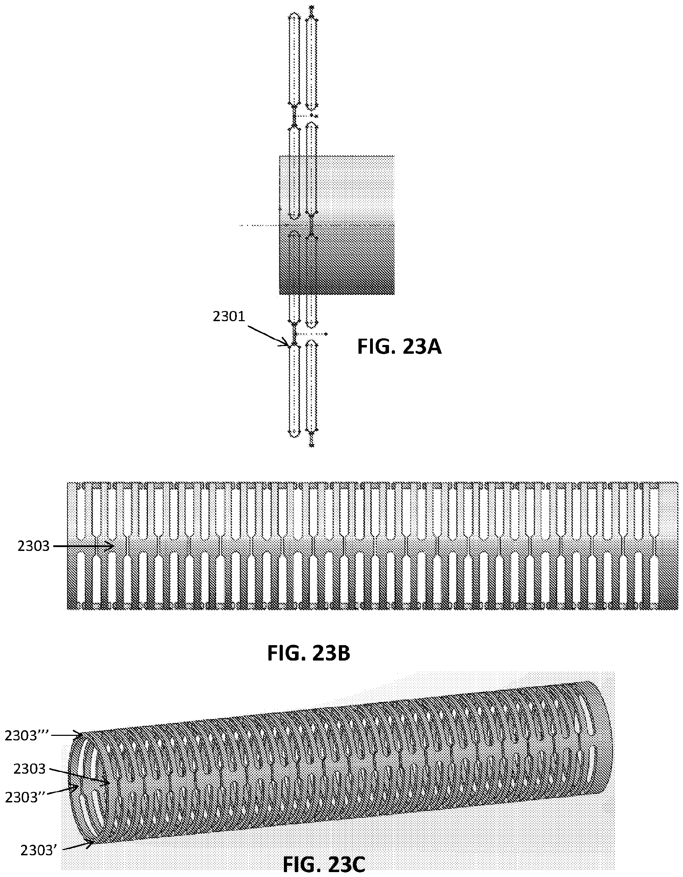

FIGS. 23A-23C illustrate another example of a slotted catheter having a dog bone pattern of aligned slots. FIG. 23A shows the repeating cut-out pattern that may be applied to a catheter (e.g., nitinol catheter tube). FIG 23B shows a side view of the slotted catheter. FIG. 23C shows a perspective view of the slotted catheter.

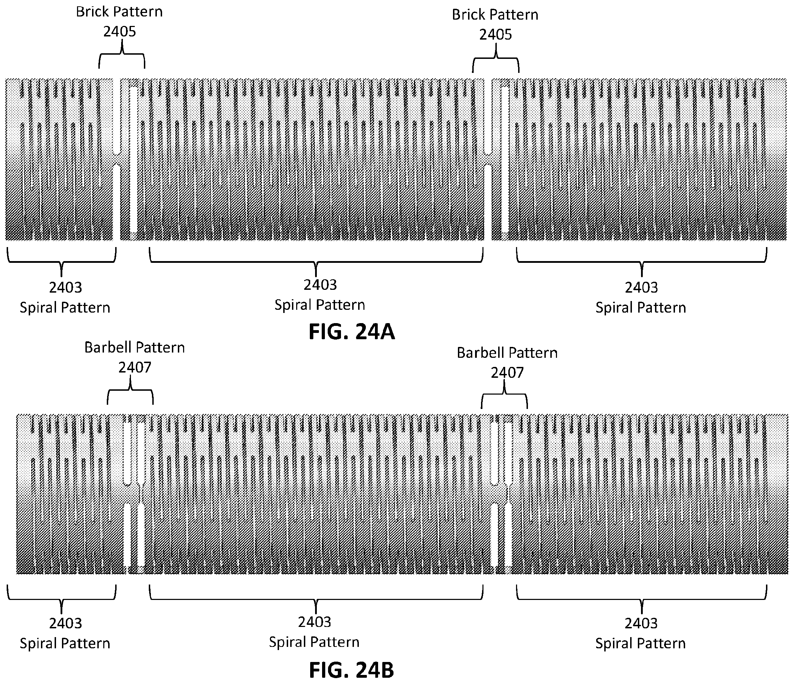

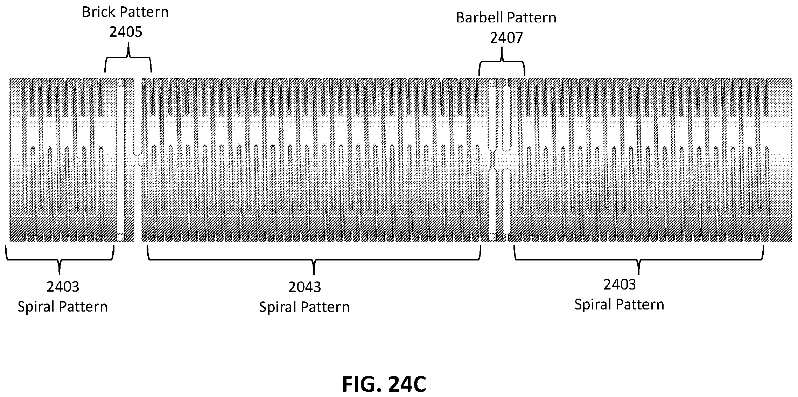

FIGS. 24A-24C illustrate slotted catheters having different slot patterns along their length. FIG. 24A shows an alternating spiral/brick pattern. FIG. 24B shows an alternating spiral/dog bone pattern. FIG. 24C show a pattern of spiral/brick/spiral/dog bone/spiral, which may also alternate.

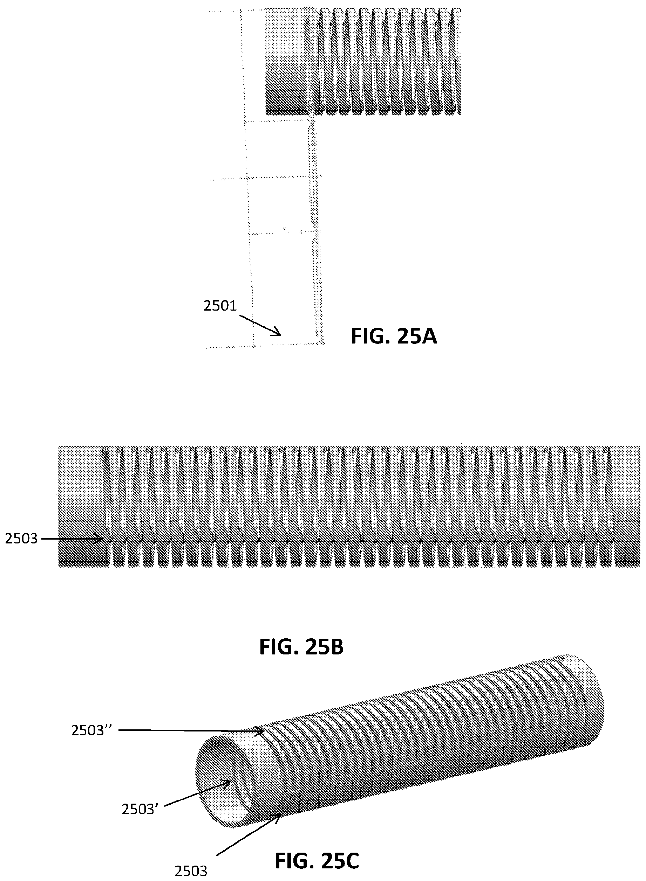

FIGS. 25A-25C illustrate an example of a slotted catheter having a continuous spiral slot cut into the catheter body. FIG. 25A shows the cut (slot) pattern including a notched, "bump" region that is spaced so as to align with a series of notched bumps in the long axis of the catheter, when cut in the spiral pattern, as illustrated in FIGS. 25B and 25C. In FIG. 25B a side view of the catheter is shown. FIGS. 25C shows a side perspective view of the catheter of FIGS. 25A-25C. Although three longitudinal lines of aligned bumps are sown circumferentially spaced in FIGS. 25B-25C; any number of aligned bumps (e.g., greater than 2, between 1 and 60, etc.) etc.) may be formed by the spiral cut.

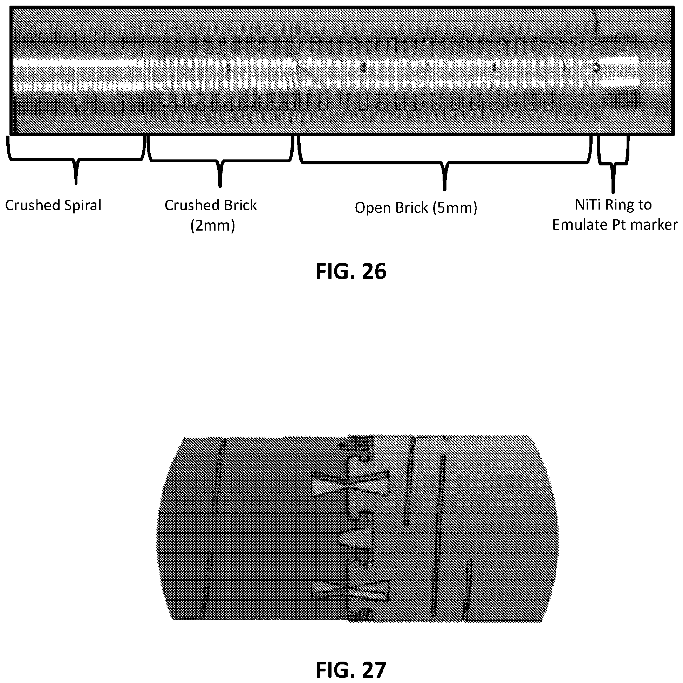

FIG. 26 illustrates one example of an elongate slotted inversion support catheter of a mechanical thrombectomy apparatus for removing a clot from a vessel formed of both a stainless steel (laser cut) and NiTi (nickel titanium) that has been shape-set.
FIG. 27 illustrates a method of joining two regions of an elongate slotted inversion support catheter that are formed of different materials (e.g., stainless steel and nickel titanium).

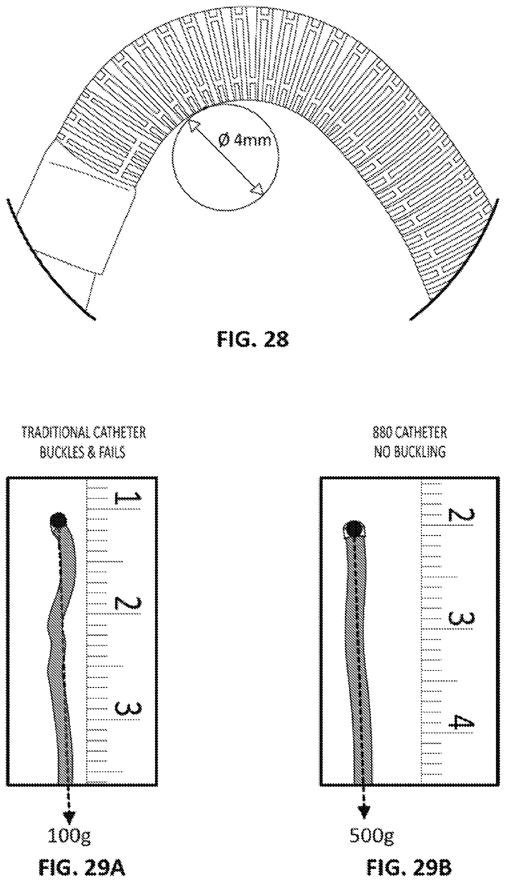

FIG. 28 is an example illustrating the tracking (bending) of a distal end region of an elongate slotted inversion support catheter.
FIG. 29A illustrates an example of a traditional catheter exposed to a high compression force (e.g., when pulling a guidewire through the distal end of the apparatus), showing buckling along the length.
FIG. 29B is another example illustrating an apparatus configured as described herein, e.g., using a specifically configured elongate slotted inversion support catheter having a region of the elongate inversion support catheter comprising a plurality of slots arranged approximately transversely to a long axis of the elongate slotted inversion support catheter, wherein there are between 2 and 4 slots per circumferential turn about the long axis.

### DETAILED DESCRIPTION

In general, disclosed and described herein are mechanical thrombectomy apparatuses having an inverting flexible tractor tube that is configured to invert over an elongate inversion support catheter. One end of the tractor tube may be coupled to a puller (e.g., pull wire, pull catheter, etc.). In particular, disclosed and described herein are apparatuses including one or more adaptations to prevent jamming and/or roll smoothly, particularly when pulling a large and/or hard clot.

In general, a mechanical thrombectomy apparatus for removing a clot from a vessel may be a system, assembly or device including an elongate inversion support catheter having a distal end and a distal annulus (distal end opening), and a flexible tractor assembly including a flexible tractor tube coupled to an elongate puller. The flexible tractor tube is configured to roll and invert over the distal end opening of the elongate inverting support catheter.

In many of the examples disclosed and described herein, the tractor assembly is configured to extend within the elongate inversion support catheter when deployed. Any of these apparatuses may switch between a delivery configuration, e.g., in which the entire tractor assembly may be held within the elongate inversion support catheter prior to deployment, and a deployed configuration, e.g., in which the elongate inversion support catheter is positioned between the flexible tractor tube and the elongate pusher to support the flexible tractor tube as it is pulled into the elongate inversion support catheter distal end opening to roll and invert into the elongate inversion support catheter. In particular, the methods and apparatuses may be configured so that the transition between the delivery configuration and the deployed configuration is robust. For example, as will be described in greater detail herein, any of the apparatuses and methods disclosed and described herein may include an annular bias that enhances the ability of the elongate inversion support catheter to be inserted between the flexible tractor tube and the elongate puller.

FIGS. 1A to 1I illustrate various components of a mechanical thrombectomy apparatus that may include any of the features disclosed and described herein. For example, FIG. 1A shows a catheter (e.g., an elongate inversion support catheter) that may form part of the apparatuses disclosed and described herein. In this example, the elongate inversion support catheter includes a catheter body 100 having a distal end region 103 that includes a distal end opening 105. The distal end region may have an increasing softness (measured by durometer, e.g., shore durometer) except that the very distal-most end region (distal end 105, including the distal end opening) may be substantially less soft than the region immediately proximate to it. Thus, although the distal tip region of the catheter (e.g., the distal most x linear dimensions, where x is 10 cm, 7 cm, 5 cm, 4 cm, 3 cm, 2 cm, 1 cm, 9 mm, 8 mm, 7 mm, 6 mm, 5 mm, 4 mm, 3 mm) has an increasing softness/decreasing harness extending from the proximal to distal ends, the very distal end region 107 (e.g., measured as distal most z linear dimensions, where z is 1 cm, 9 mm, 8 mm, 7mm, 6 mm, 5 mm, 4 mm, 3 mm, 2 mm, 1mm, 0.8 mm, 0.5mm, 0.3 mm, 0.2mm, etc., and z is always at least three times less than x) has a hardness that is greater than the hardness of the region immediately proximal to it, and may be as hard or harder than the proximal-most region of the distal tip region.

In FIG. 1A, the elongate inversion support catheter is an elongate hollow catheter having a column strength that is sufficient to prevent buckling when the catheter is pulled over the distal annulus (distal end opening). Thus, the elongate inversion support may be configured so that it does not collapse (e.g., buckle) when 500 g or less of compressive force is applied (e.g., at least about 700 g, 600 g, 500 g, 400 g, 300 g, etc. of compressive force) for neurovascular applications. For peripheral vascular applications the elongate inversion support may be selected or configured to withstand at least 1500 g of compressive force (e.g., at least about 2000 g, 1900 g, 1800 g, 1700 g, 1600 g, 1500 g, 1400 g, etc. of compressive force). In general, any of the apparatuses disclosed and described herein may include an elongate inversion support catheter that is not a full-length catheter, but may include a portion of a catheter, typically at the distal end, connected to a rod, wire, hypotube, or the like. In FIG. 1A the catheter 100 of the elongate inversion support catheter may be any appropriate type of catheter or portion of a catheter, including microcatheters appropriate for neurovascular use.

In some variations the distal end 105 of the elongate inversion support is adapted so that the tractor may slide or roll and invert over the distal end of the catheter without being caught (binding, jamming) or without substantial friction. For example, in some variations the distal tip (end) may be curved or radiused 109 as shown in FIG. 1B, particularly on the outer surface (e.g., the transition from outer diameter to inner diameter).

FIG. 1C shows an example of a flexible tractor tube 144 coupled to an elongate puller 146, forming a pullable tractor assembly 140. In this example, the tractor tube is shown integrated with the puller and extending back over the puller, forming the assembly. The opposite end of the flexible tractor tube 147 is open and free (e.g., not connected to the puller or catheter). As will be described in greater detail below, this open, free, end may be adapted to be expanded and held open, e.g., by shape setting back on itself and/or by including an annular bias, to enhance deployment and positioning of the catheter between the flexible tractor tube and the puller. In FIG. 1C, the tractor tube is formed of material (e.g., wove, knitted, braided, etc.) that is flexible and elongate. The tractor is shown extended from the puller in a first configuration. It may be particularly beneficial if the relaxed outer diameter of the flexible tractor in this first configuration has a greater outer diameter than the outer diameter of the catheter of the elongate inversion support into which the tractor will be positioned prior to inverting. The flexible and tubular tractor 144 may be sufficiently soft and flexible (e.g., having a low collapse strength) so as to easily roll and fold over the distal aperture of the elongate inversion support. The puller 146 may typically be a less-expandable (or non-expandable) structure (tube, puller, etc.). For example, the tractor 144 may be configured, e.g., by shape-setting (heat setting, etc.), to expand in the relaxed first configuration to a radial diameter that is between 1.1 and 10 times (e.g., between 1.1x and 5x, between 1.1x and 4x, etc.) the diameter of the inner diameter of the catheter of the elongate inversion support when unconstrained. In FIG. 1D, the tractor tube has a larger expanded diameter than the variation shown in FIG. 1C in a relaxed configuration. In any of these variations, the expandable tractor may be biased to expand open. The tractor may be formed of a mesh, braided, woven, knitted, or sheet of material and is generally adapted to grasp the object to be removed (e.g., blood clot).

In FIGS. 1C and ID the tractor and puller have two portions, a tractor tube 144 and a less expandable (or non-expandable) proximal portion comprising the elongate puller 146. The puller may be a separate region, such as a wire, catheter or hypotube, which is connected to an end region of the tractor (e.g., a flexible mesh, woven, braided, etc.), e.g., the distal end or near the distal end. The inverting region of the tractor, where it rolls and inverts over the distal end opening of the catheter may be referred to as the distal-facing region of the tractor, which may actively grab clot when rolling.

In FIG. IE, the tractor assembly (flexible tractor tube 144 and puller 146 of FIG. 1D) are shown within an elongate inversion support catheter 100. The tractor is collapsed down 101, e.g., onto the puller, and may be held collapsed within the elongate inversion support catheter. Thus, FIG. 1E shows the pre-deployment (e.g., delivery) configuration. The tractor assembly may be axially movable (slidable) within the catheter so that it can be positioned within the catheter and within the vessel.

FIG. 1F shows a fully deployed apparatus. In FIG. IF, the tractor tube is in an unconstrained or deployed configuration, and the elongate inversion support catheter is positioned between the tractor tube and the puller so that the tractor tube can be pulled proximally by pulling on the puller and rolling the tractor tube into the elongate inversion support catheter so that it inverts. In FIG. IF, the tractor in this deployed configuration (e.g., the portion that is inverted over the distal end of the catheter) has an outer diameter that is greater than the outer diameter of the catheter of the elongate inversion support. Thus, the tractor 144 may be biased so that it has a relaxed expanded configuration with a diameter that is greater than the outer diameter (OD) of the elongate inversion support catheter; in addition, as will be described in relation to FIGS. 1G and 1H, below, the tractor tube may also be configured (e.g., by heat setting, etc.) so that when the tractor tube is inverted and pulled into the elongate inversion support catheter, the outer diameter of the inverted tractor tube has an outer diameter that is greater than 0.5x (e.g., greater than 0.6x, greater than 0.7x, greater than 0.75x, greater than 0.8x, greater than 0.9x, greater than 1x, etc.) the inner diameter (ID) of the elongate inversion support catheter. This combination of an un-inverted diameter of the tractor tube of greater than the diameter of the OD of the elongate inversion support catheter and an inverted diameter of the tractor tube of greater than 0.7x the ID of the elongate inversion support catheter is surprisingly helpful for preventing jamming of the apparatus, both when deploying the apparatus and when rolling the tractor over the distal end opening of the elongate inversion support catheter to grab a clot. The tractor may be expandable and may be coupled to the puller as shown. In some variations the flexible tractor and the puller may comprise the same material but the tractor may be more flexible and/or expandable, or may be connected to elongate puller (e.g., a push/pull wire or catheter).

FIGS. 1G and 1H illustrate the removal of a clot using an apparatus such as the apparatus components of FIGS. 1A and 1E. The apparatus 10 is shown in a deployed state. In this example the thrombectomy apparatus 10 is configured as a thrombectomy apparatus including an elongate inversion support catheter 100 and a flexible tractor tube 144 that extends over the distal end region of the catheter and doubles-over itself at the distal end of the catheter to invert so that the external tractor end region is continuous with an inner, less-expandable (in this example, less-expandable includes non-expandable) second distal end region 146 (puller) that extends proximally within the catheter and forms an inner lumen that may pass a guidewire. The pusher/puller member that may be a rod or other member that is continuous with the distal end region of the tractor. In FIG. 1G the apparatus is shown positioned and deployed within the vessel 160 near a clot 155. The clot may be drawn into the catheter by pulling the tractor 140 proximally into the catheter 101, as indicated by the arrow 180 showing pulling of the inner portion of the flexible tractor (e.g., using a handle, not shown) resulting in rolling the tractor over the end opening of the catheter and into the catheter distal end and inverting the expandable distal end region so that it is pulled into the catheter, shown by arrows 182. The end of the tractor outside of the catheter may be "loose" relative to the outer wall of the catheter. FIG. 1I illustrates another example of a tractor assembly 154 including a tractor tube 144 that is coupled to a puller 156. The puller in this example is tapered (having tapering region 161) and may therefore have a different flexibility of the distal end region than the proximal end region. For example, the proximal end region may be less flexible than the narrower-diameter distal end region 195 to which the tractor is coupled. The assembly includes a radiopaque marker 165. The tractor may be attached to the puller by any appropriate means. For example, the tractor may be crimped, glued, fused, or otherwise attached to the puller, typically permanently.

In general, the mechanical thrombectomy apparatuses disclosed and described herein may be highly flexible, both before actuating and during operation. For example, the flexible tractor may not increase the stiffness/flexibility of the catheter of the elongate inversion support, and particularly the distal end region of the catheter too much, to avoid impacting maneuverability, particularly within tortious vessels of the neuro-vasculature. Disclosed and described herein are flexible tractor tube portions that increase the stiffness of the last y cm (e.g., distal most 20 cm, 18 cm, 15 cm, 12 cm, 10 cm, 9 cm, 8 cm, 7 cm, 6 cm, 5 cm, 4 cm, 3 cm, 2 cm, 1 cm, etc.) of the catheter less than a predetermined percentage (e.g., less than 10%, 12%, 15%, 18%, 20%, 25%, 30%, etc.). For example, disclosed and described herein are flexible tractor tube portions that pass through the catheter and double back over the distal end of the catheter but increase the stiffness of a distal 5 cm of the catheter by less than 15% of the stiffness of the distal 5 cm of the catheter without the flexible tube extending therethrough and doubling back over the distal end of the catheter.

The tractors may be woven, braided and/or knitted materials. For woven and braided materials, which may include a plurality of fibers that are woven or braided to form the inverting tube, these structures may be tuned to prevent jamming and/or to reduce the force necessary to pull the tractor and invert over the catheter tip. For example, the mechanical atherectomy apparatus may include a braid-type tractor that can roll freely around the tip of catheter even in a tortuous anatomy and when grabbing clot by tuning one or more of the braid structure; minimizing the braid angle; including a hydrophilic coating on the distal aspect of the catheter outer diameter (OD) or the inner diameter (ID) of the braid (e.g., tractor); including a radiused wall on the catheter; and/or increasing the stiffness of the distal tip region relative to adjacent proximal regions. Alternatively, it may be advantages to have a hydrophilic coating on 1, 3,5,10, or 15 cm of the distal ID or the entire catheter ID. This may even enhance aspiration of the clot without a tractor element.

As mentioned, the tractor (e.g., braided, woven, knitted, etc.) may be configured to collapse down into the inner diameter (ID) of the catheter as little as possible. For example, the tractor may collapse to an ID that is greater than, equal to, or within 90%, 85%, 75%, 70%, 65%, 60%, or 50% of the catheter inner diameter (ID)/Catheter Tip OD, since, when the tractor is being pulled around catheter tip it may create axial tension on the tractor (e.g., braid, knit, etc.) that can inadvertently cause the tractor to jam on the catheter tip. When tractor is pulled around catheter tip, the tractor is being pulled in the axial orientation creating axial tension on tractor structure as the tractor is being pulled through the catheter ID. By having the tractor elements jam at an ID greater than or equal to 90%, 85%, 75%, 70%, 65%, 60%, or 50% of the catheter ID (or in some variations, OD), when being axially tensioned, the tractor is less likely to grab/synch down onto the catheter tip, helping the braid roll around the catheter tip with less axial force applied by the user. If less axial force is required by the user to pull the tractor structure around the tip then the catheter tip is less likely to buckle or deflect when retracting the tractor. It may be advantageous to minimize the chance the catheter tip will buckle. The tractor can be tuned to "jam" at a specific ID by controlling any of the following variables and in any combination: selecting a specific number of braid ends, selecting the size/diameter of the braid ends; selecting the braid material (e.g., multifilament or monofilament); heat setting the bias on the braid (e.g., braid diameter); and selecting a braid pattern, e.g., 1x2, 1x1 or any other pattern.

The braid angle may be minimized to prevent locking up of the rolling of the tractor over the catheter end opening. Typically, the lower the braid angle (e.g., 45 degrees or less, 40 degrees or less, 35 degrees or less, 30 degrees or less, 25 degrees or less, 20 degrees or less, etc.) the less likely it is to have the braid cross over points catch on the catheter tip.

In any of the variations disclosed and described herein, the catheter and/or a surface of the tractor may be coated to enhance rolling over the distal end region of the catheter. It may be helpful to have a hydrophilic coating on the distal aspect of the catheter OD or the ID of the tractor so the tractor can more easily side over the catheters distal end and around the tip of the catheter when pulled through the inside of the catheter.

The radius wall of the catheter tip may be chosen/set to within a range that allows sliding. For example, it may be helpful for the tip of the catheter to have the largest radius possible but at least 0.0635mm (0.0025") radius wall on the catheter, ideally approximately 0.127mm (0.005") radius wall.

The stiffness of the distal of the catheter may be sufficiently stiff to prevent collapse as the tractor is pulled; it may also be lubricious (e.g., by a coating or material property). The distal most section of the catheter tip (e.g., the last 5mm) may be fabricated of a material which is stiff enough and lubricious enough so the distal tip of the catheter does not collapse or buckle inward ward when the braid structure is rolling around the catheter tip. Thus, the distal tip may have a stiffness that is greater than the more proximal region at the distal end of the catheter.

It may be helpful or desirable to have pores in the tractor. A lack of gaps or small pore size may limit the ability of the braid to grab clot. Alternatively, or additionally, it may be desirable to form a braid structure with texture. One example is to braid two or more different diameter braid ends into the same structure: the difference in braid end diameters will help form a texture to the braid structures outer surface, aiding the grabbing of the clot when rolling the braid-dozer around the catheter tip.

As an alternative (or in addition) the tractor may be configured to lock so it does not compress in diameter during axial load by adding a coating, laminate or adhesive to the braid at a desired diameter. Adding a thin coating, laminate or adhesive can inhibit the braid elements from sliding with respect to each other, thereby locking the braid to a specific diameter. The coating can be applied while leaving the majority of the pores and pore area substantially open. Examples of thin coatings include urethanes and silicones with and without hydrophilic coatings and hydrophilic coatings with no tie layer.

Reducing the sliding friction of tractor to outer catheter wall, improving tractor to tip rolling, and/or enhancing tractor to inner catheter sliding may also be achieved by including a sliding skin or sleeve. For example, a thin (e.g., ultrathin) sleeve may be used. The sleeve would be made from low friction polymer (e.g., PET, PE, PP, PTFE, ePTFE, pebax, urethanes) by braiding, knitting, weaving, extrusion, melt blown, melt spinning, etc. The sleeve could be made from laser slotted tubing, chemical etching, micro machining. The sleeve could be also coated with a lubricious coating such as a hydrophilic coating. Lubricious coatings can be located on the outside and/or inside surfaces. The sleeve may be placed between the dozer element and the catheter wall and attached to the puller element. The sleeve may be less than 0.0508mm (0.002") thick, ideally, less than 0.0254mm (0.001") wall thickness. The sleeve may decouple the tractor clot grabbing system from the catheter wall, tip rolling and inner catheter dragging friction. The sleeve could be totally free from the tractor, connected to the tractor in discrete locations or connected fully to the tractor. This may allow the tractor to be designed to grab clot (larger wires: 0.0254mm (0.001") to 0.0508mm (0.002") for neuro, and 0.0508mm (0.002") to 0.1778mm (0.007") for other applications) and the skin to minimized in thickness and structure to reduce friction and skin bending stiffness.

In any of the apparatuses disclosed and described herein, the catheter (e.g., the inversion support catheter) may be covered with a jacket along all or a part of its length. This jacket may be highly elastic, and may include a single layer or multiple layers. The jacket may be have a single durometer (e.g., stiffness) or multiple durometers along its length; for example the jacket durometer may be less stiff near the distal end of the catheter. The jacket may be formed of any appropriate material, including urethane (e.g., Teflon 20A to 93A, e.g., 80-85A), PEBAX (e.g., 25A to 72D), silicone, nylon, etc. In an of these variations the jacket may be continuous or formed along with a sleeve such as described above, that transitions from the inside of the catheter and around this distal end opening and along the outer surface of the catheter. The jacket may be integrally formed and/or fused to the sleeve. The sleeve may also be referred to as a liner. As mentioned, the sleeve may be a lubricious material, such as PTFE, FEP, HDPE, Polypropylene, and/or other polymers, particularly those with modifier such as siloxane modifiers. The jacket may be bonded to the sleep/liner (e.g., through the cut frame of the catheter, such as a slotted catheter).

In some variations, the tractor region may be formed of with a mixed or hybrid structure, combining one or more of interwoven or knitted braid polymer filaments with metallic filaments. The mixed structure (hybrid structure) may leverage both metallic elements interwoven with low friction polymer elements. The metallic filaments may create stiffness elements that may grip/grab a clot. The polymer filaments may aid in grabbing clot but may provide surface friction reduction to the outer catheter wall, the catheter tip and the inner catheter wall once around the tip.

Any of the apparatuses disclosed and described herein may include a tractor having a hydrophilic/lubricous coating on the inside surface, e.g., for braided/knitted tractors, on the inside surface (contacting the outer and inner diameter of the catheter) of the braid/knit, which is in contact with the outside of the catheter. Examples of lubricous coatings include hydrophilic coatings (e.g., hydrogels) and hydrophobic coatings (e.g., fluorine coating such as PTFE & FEP, parylene, silicone, siloxane (silicone additive) added to various polymers including pebax to make any material more lubricious, Polyethylene, polypropylene, FEP)

As mentioned above, any of these apparatuses may include a distal tip that is less rigid (e.g., 'softer') than the more proximal regions of the distal tip. This may be achieved by having a structural supporting member reinforcing the distal tip, or by modifying the material forming the distal tip.

Any of the tractors disclosed and described herein may include a marker or makers (e.g., radiopaque markers, such as gold, Pt, etc.).

Any of the apparatuses disclosed and described herein may include a lubricious sleeve within the distal end of the elongate inversion support catheter to enhance rolling of the tractor tube, and particularly a knitted tractor tube into the distal end of the elongate inversion support catheter. For example, FIG. 1A illustrates a first example of a mechanical thrombectomy apparatus including a lubricious liner 211 that wraps over the distal end of the elongate inversion support catheter 201. In this example, the inverted knitted tractor tube 203 rolls over the distal end of the elongate inversion support catheter and is attached at one end to a puller 207 (shown as a pull wire in this example). Pulling proximally on the puller rolls the tractor tube into the elongate inversion support catheter's distal end opening and pulls the clot 219 into the elongate inversion support catheter. The apparatus may include or may be operated with a guide catheter 205, so that the inner elongate inversion support catheter may be extended distally out of the guide catheter. In some variations, the guide catheter may be used to releasably secure the outer portion of the tractor tube 203 against the outer surface (outer diameter) of the elongate inversion support catheter 201 and may be retracted fully (as shown in FIG. 2A) or partially (as shown in FIG. 2B) when operating the apparatus to grab and/or remove a clot 219.

FIG. 3 schematically illustrates an exemplary method of removing a clot using an apparatus including a lubricious sleeve inverting over the distal end of the elongate inversion support catheter, similar to that shown in FIG. 2A. In FIG. 3, the method may include generally delivering a guide (or "intermediate") catheter to the face of a clot 301. This step may be optionally performed with the mechanical thrombectomy apparatus (e.g., the elongate inversion support catheter and tractor tube pre-loaded, e.g., near the distal end of the delivery catheter). Thereafter, the thrombectomy catheter may be delivered thru guide (intermediate) catheter 303 so that the elongate inversion support catheter is near the face of the clot. Once in position, the tractor may be deployed; for example, while applying some push force on the elongate inversion support catheter, the guide (intermediate) catheter may be retracted to un-sheath the tractor tube 305. This may result in the arrangement shown in FIG. 2A, for example. Optionally, aspiration may be applied through the elongate inversion support catheter (and/or the puller in variations of the puller having an inner lumen) before retracting the guide (intermediate) catheter. Aspiration may therefore be optionally through elongate inversion support catheter (and/or the intermediate catheter). Thereafter, the puller connected at one end to the tractor tube may be pulled proximally, preferably while putting forward an advancing force on the elongate inversion support catheter, to grab and remove the clot from the vessel 309.

Alternatively, as shown in FIG. 2B, the guide (intermediate) catheter 205 may be only partially withdrawn over the elongate inversion support catheter 201 so the distal end of the knitted tractor tube 203 is still contained inside the intermediate catheter. This configuration may provide additional support for the elongate inversion support catheter when engulfing the clot with the knit tractor tube, so it is less likely to buckle or snake when it is exposed to the related compressive loads.

This configuration may also, because of the additional outside support of the intermediate catheter, allow the user to more easily advance the elongate inversion support catheter forward in the vessel as the trailing edge of the clot is engulfed. The intermediate catheter can cover 10%, 20%, 30%, 40%, 50%, 60%, 70%, 80%, 90%, etc., of the tractor length on the outside of the catheter. Or the intermediate catheter can be positioned less than 1 cm, 2 cm, 3 cm, 4 cm, 5 cm, 10 cm, etc. back from the distal tip of the elongate inversion support catheter.

The lubricious liner wrapping around the elongate inversion support catheter tip may be any appropriate liner. The liner typically wraps from an outer surface of the elongate inversion support catheter over to an inner surface of the elongate inversion support catheter. This may be in addition to or instead of a coating and/or shaped distal end region of the elongate inversion support catheter.

FIGS. 6A and 6B illustrate another example of a lubricious liner. For example, in FIG. 6A the lubricious liner 601 is a PTFE liner wrapping around the open distal end of the elongate inversion support catheter 603. A tractor tube (a nickel titanium knitted tractor 605) is shown inverting into the end and over the PTFE liner 601. One end of the tractor tube is connected to a puller 609 and may be drawn proximally to pull in a clot 607.

Alternatively, or additionally, the elongate inversion support catheter may be shaped to reduce the pull force need to to invert the tractor tube into the elongate inversion support catheter. For example, FIG. 6B shows an elongate inversion support catheter 613 distal end opening may be shaped (e.g., chamfered 645) to reduce the pull force/friction.

In general, larger (e.g., clots having a diameter greater than 2x the diameter of the elongate inversion support catheter) and/or hard clots may be more difficult to compress and pull into a mechanical thrombectomy apparatus. Surprisingly, the apparatuses and methods disclosed and described herein may be used to draw even larger and hard clots into the apparatus.

A hard clot typically may take a significant compressive load in order to compress the clot and engulf it in a smaller ID elongate inversion support catheter. As mentioned, hard clot may require a significant compressive load to compress the clot, generating a relatively high normal force to the distal & inner end of the catheter wall. The low friction surface (including sleeves) disclosed and described herein may help reduce the load to pull in clot to less than 300 g of force, when measured from the pull force required to pull the tractor grabbing the clot into the elongate inversion support catheter (e.g., less than or equal to 300 g, less than or equal to 250g, less than or equal to 200 g, etc.). FIG. 4A and 4B illustrate the compression of a large-diameter (compared to the diameter of the elongate inversion support catheter) clot into an apparatus as disclosed and described herein. FIG. 4A shows a 3mm clot being pulled into a 0.045 ID catheter (having a diameter of 1.22 mm). In FIG. 4B, the arrows 403 show normal forces generated at the inner opening edge of the elongate inversion support catheter.

As mentioned above, any of the apparatuses disclosed and described herein may preferably include knitted tractor tubes. The orientation (e.g., "inside out") as well as the shape-set of the knit may be selected to prevent jamming, reduce the pull force necessary to invert the tractor into the elongate inversion support catheter, and may help knead, compress, and/or drive the clot into the opening of the elongate inversion support catheter. For example, FIG. 5 illustrates an example of a knitted tractor 501 being pulled to invert into an elongate inversion support catheter 507. The knit is formed by knitting single strand of wire (though multiple strands of one or more filaments, weaves, braids, etc. may be used) to form a plurality of rows of loops 503 that are interconnected. The loops may be pulled and inverted into the distal end opening of the elongate inversion support catheter so that, when in the inverted knit configuration, they flare outward and push the clot 505 into the elongate inversion support catheter. In FIG. 5 the clot is a large-diameter clot (>2x the diameter of the elongate inversion support catheter) and the knit tractor is shown flaring out to whip in front of the elongate inversion support catheter tip to knead the clot into catheter.

FIGS. 7A-7F illustrate an example of knitted tractor tubes. FIGS. 7A (and the enlarged view 7B) illustrate a first variation of a knitted tractor tube having a knit pattern that is less optimal, as the diameter of the knitted tractor tube is sufficiently large so that there are triple crossover points between the wire (e.g., strand, fiber, filament, etc.) forming the knit. Such "triple overlap" regions 705 may result in a thickness or OD of the tractor tube that is undesirably large. Thus, it may be better to size and/or shape set the knitted tractor tube to prevent or minimize these triple overlap regions in the links forming the knit (e.g., having less than 10% triple overlaps, <9%, <8%, <7%, <6%, <5%, <4%, etc.). FIGS. 7C (and the enlarged region shown in FIG.7D) illustrate a more optimized variation of a knitted tractor tube showing only double overlaps 707.

As mentioned above, it is also desirable to use a knitted tractor tube pattern in which the orientation of the knit is inverted (as compared to most commercially available knitted tubes), so that the links, when the tractor tube is inverted over the distal end opening of an elongate inversion support catheter and pulled into the elongate inversion support catheter, the links flair outward. Thus, the orientation of the knit may be configured so that the apex (tip) of the loops in each row are on the outside face of the elongate inversion support catheter over which the tractor tube is inverting, compared to the adjacent pair of links that each link loop is connected to. This inverted knit configuration is shown in FIG. 7C and 7D. For example, in FIG. 7D the link 703 is formed of a filament (e.g., nitinol, polymeric material, etc.) that is knitted into a pattern of adjacent links that are interconnected (e.g., as the double crossover points 707, 707'). The knitted tractor tube is shown over an elongate inversion support catheter 709, so that the outward-facing surface of the knitted tractor tube includes all of the apexes 711 of each link. These links are therefore arranged to pass under then over a first adjacent link, then over and under a second adjacent link, as shown in FIG. 7D, leaving the apex region on the outward-facing side of this "inverted" knit configuration. In contrast, FIGS. 7E (and enlarged view 7F) show a non-inverted knit configuration, in which each link 713 includes an apex 721 that is instead underneath (on the side facing the elongate inversion support catheter, not shown). Each loop is this example is formed of a filament that is woven in an over-under and then under-over pattern relative to adjacent links of the knit. The non-inverted knitted tube shown in FIGS. 7E and 7F is constrained when inverting over an elongate inversion support catheter, so that it cannot swing outward relative to the distal end of the elongate inversion support catheter, by the adjacent links. As a result, the pulling force of the tractor tube is surprisingly higher, and the clot-grabbing of the tractor may also be greatly reduced.

FIG. 8A shows an example of an inverted knit tractor tube rolling over the distal end opening of an elongate inversion support catheter. In any of these variations, the knitted pattern may be optimized so that the stich length (L) may be tuned to get a desired grab width (W) increase that is greater than elongate inversion support catheter outer diameter (OD). This larger grab width may aid in grabbing a clot with the apparatus. In FIG. 8A, the length of each knitted link (L) is shown, as is the width (W) that the link ends outwards when inverting into the itself and the elongate inversion support catheter. Typically, it has been found to be desirable to have a width (W) of between about 30-90% of length (L). The exact dimension of the width (W) may depend on the tension in the knit used to pull in the clot (e.g., the great the tension, the smaller the W dimension), and the flexibility of the filaments used in the knit structure (e.g., the softer the filament used to form the knit, the smaller the W). The knit can be tuned through both the design and the processing so when it is tensioned at loads between 100-500 grams (g), the structure may have a limited elongation (e.g., <15%, <12%, <10%, etc.0). When the knit structure is in this non-compliant/semi-compliant form the OD & ID of the knitted tractor tube may also be stable (e.g., having a <20%, less than 15%, less than 12%, less than 10%, etc. diameter change) under loads between 100-500 g. In this non-complaint/semi compliant form, the knit OD may be greater than the thrombectomy catheter, but less than the vessel OD. In other variations, the knit OD may be greater than the catheter OD, and the knit is rolled around and less than 5%, %10%, 20%, 30%, 40%, 50%, 60%, 70%, 80% of the same catheter OD.

Also disclosed and described herein are reusable mechanical thrombectomy apparatuses. For example, FIGS. 9A-9C illustrate one reusable mechanical thrombectomy apparatus and method of use. In FIG. 9A, the apparatus includes a tractor tube (shown here as a knitted tractor tube 903). One end of the tractor tube is coupled to a puller 909 within the inner diameter of the elongate inversion support catheter 905. The puller in this example is configured as a hollow tube, which may allow passing of a guidewire and/or vacuum. The other end of the tractor is coupled to a distal tractor stop forming a finger grip 901 that is configured to slide over the outer surface of the elongate inversion support catheter. The finger grip may be sized for ease in grabbing manually and sliding proximally to "reload" the apparatus, as described below.

In FIG. 9A, the apparatus may be positioned near the clot 911, and the puller drawn proximally to invert and roll the tractor tube 903 into the elongate inversion support catheter 905, while advancing distally, and thereby pull the clot 911 into the elongate inversion support catheter (shown by arrow. 917). Once grabbed, either fully or partially, as shown in FIG. 9B, the apparatus may be withdrawn from the vessel, the clot may be removed, as shown in FIG. 9C, by pulling the outer portion of the tractor tube proximally. The tractor tube is prevented from completely inverting into the elongate inversion support catheter by a distal stop 907 on the elongate inversion support catheter. For example, in FIG. 9C, the outer portion of the tractor tube may be manually pulled proximally 915 by sliding the distal tractor stop/grip 901 proximally, ejecting the compressed clot 911 from inside the apparatus. The device may then be rinsed and re-used to remove additional clot(s).

Any of the apparatuses disclosed and described herein may be adapted to enhance tracking with an intermediate catheter and/or a lumen of a vessel. To assist the mechanical thrombectomy apparatus in tracking inside an intermediate catheter or tracking directly inside a native vessel (e.g., in which the puller, tractor tube and elongate inversion support catheter must track inside the distal end of an intermediate catheter), it may be beneficial to allow the puller to extend distally relative to the elongate inversion support catheter without disrupting the tractor tube. For example, FIG. 10A illustrates one example of a mechanical thrombectomy apparatus according to the invention, including a tractor tube 1003, elongate inversion support catheter 1005 and puller 1007, which is shown within an intermediate catheter 1010. In this variation, the distal end of the puller is configured to include a bumper region 1009 that can extend distally out past the distal end of the elongate inversion support catheter 1005. This nose element ("bumper") 1009 may be a constant stiffness or variable stiffness structure. For example, the distal end may be the softest section and the section most proximal to the distal end of the catheter may be less soft, and/or possible tuned to be of similar stiffness to the distal end of the elongate inversion support catheter. The tractor tube may be coupled proximal to the distal end, which may extend, e.g., approximately 1 cm, as shown.

The distal nose of the pull wire is cannulated. A cannulated nose allows for guidewire access and may also allow for aspiration through the intermediate catheter to allow vacuum forces to reach any clot or any other targeted element located in the body or vessel.

In use, the user may approach the clot within the lumen of the vessel with the nose region of the puller extended from the elongate inversion support catheter or extended within the elongate inversion support catheter. Once near the clot the nose region may be retracted inside the elongate inversion support catheter. Next the user may optionally pull a vacuum through the elongate inversion support catheter to engage the clot into the tip of the elongate inversion support catheter. Then, while slightly advancing the intermediate catheter, the tractor tube may be pulled proximally into the elongate inversion support catheter by pulling proximally on the puller.

FIG. 10B illustrates another example of a mechanical thrombectomy apparatus according to the invention, having a puller (elongate puller 1027) which is configured to freely extend distally relative to the tractor tube, but when pulled proximally may engage with the tractor tube 1003 at one end to pull it proximally into the elongate inversion support catheter 1005. A portion (e.g., at or near a distal end region of the puller) may include one or more engagement regions (e.g., bumps, projections, etc.) that may engage with an annular ring or collar to pull the tractor tube proximally so that it may invert into the elongate inversion support catheter and therefore pull clot into the apparatus. In FIG. 10A, the puller can be extended beyond the tip of the elongate inversion support catheter at the user's preference when advancing the system to the clot. The male bump or other grabbing mechanism will engage on the proximal end of the tractor to pull in the clot.

FIG. 10C illustrates another variation, in which the puller 1047 is configured to pass a guidewire and maybe extended or retracted using the guidewire 1055. In FIG. 10C, the construction that allows a guidewire to track through its lumen to aid in tracking within the vessel. For example, in FIG. 10C he puller includes a guidewire channel at the distal end region to which the tractor tube is attached.

In general, any of the mechanical thrombectomy apparatuses may include a modified puller that may enhance trackability, clot grabbing and/or pulling of the tractor tube into the elongate inversion support catheter. In addition to the examples shown and described in FIGS. 10A-10C, FIGS. 11A-11D show puller apparatuses that have a distal region adapted to include one or more projections.

The puller tips shown in FIG. 11A-11D may enhance the trackability of the apparatus (e.g., the elongate inversion support catheter and/or tractor tube and/or puller) through tortuous anatomy, including the lumen of the vessel and/or an intermediate catheter. For example, FIG. 11A shows an apparatus including a tractor tube 1103, an elongate inversion support catheter 1105, and a puller 1107. In this example, the apparatus is used within an intermediate catheter 1110. The distal end 1109 of the puller 1107 is tapered and includes a plurality of projections 1115 extending away from the tapered distal end of the outer surface of the puller. In this example, the projections are configured as cups 1115 that have proximal-facing edges. The projections of this distal tip region may be used to grab and/or hook onto a clot when deployed; the puller may be advanced distally as described above and projected either by itself or over a pullwire into a clot 1127, as shown in FIG. 11A. When retracting the puller, the distal tip may pull the clot to the distal opening of the elongate inversion support catheter and/or it may advance the elongate inversion support catheter and tractor towards the clot. Any of these puller embodiments may be used with or without aspiration (e.g., through the elongate inversion support catheter, intermediate catheter and/or puller). Thus, in FIG. 11A, the distal end is configured as a cup-shaped male feature. The puller distal end region may be solid, not in accordance with the present claimed invention or cannulated and may be tapered (as shown in FIGS 11A-11D) or non-tapered (e.g., cylindrical). In FIG. 11A, each projection ("cup") extends all or partially around the entire tip and more distal projections may have smaller diameters.

Another example of a puller having a plurality of projections is shown in FIG. 11B. In FIG. 11B, the projections are configured as fibers or fingers extending proud (and may be angled proximally) from the outer surface of the distal tip off the puller 1107'. In any of these examples, the puller (including the distal tip region of the puller 1107') may be solid, not in accordance with the present claimed invention or cannulated. In FIG. 11B, the distal tip region of the puller includes one or more sets of projections 1125 (e.g., fingers or fibers) directed proximally. The projections may be constructed from an open-end braid feature. The projections may be configured (oriented and/or soft enough) to slide into the clot when extending distally, but when retracing the puller proximally the projections may flare out and help anchor the tip of the puller the clot, as shown in FIGS. 11A and 11B. Optionally the projections may also engage with the tractor tube when pulled proximally and/or distally and may help pull the tractor and clot back through the elongate inversion support catheter.

FIGS. 11C and 11D illustrate alternative variations of puller tips including protrusions. In FIG. 11C, the puller tip 1135 includes one or more helical projections 1137 extending proud of the outer surface of the puller and extending around the puller. In some variations the projections are formed of a wire wrapped around the distal tip of the puller; the wire forms the male projection. FIG. 11D shows another example of a distal tip region 1145 having a stepped profile forming projections from the distal end of the puller. In this example, the distal end is formed by a plurality (e.g., two, three, four, five, six, etc.) of cones that are arranged in progressively smaller sizes extending distally (forming a tapered tip region). The cones may be solid, not in accordance with the present claimed invention or cannulated. The tip may be formed of a plurality of connected elements or may be a unitary (or fused) element.

### INVERSION SUPPORT CATHETERS

Ad discussed above, any of the apparatuses disclosed and described herein may include an elongate inversion support catheter having a distal end and a distal end opening, in addition a tractor tube (e.g., a knitted tractor tube). In operation, the tractor tube extends over an outer surface of the elongate inversion support catheter, and inverts into the distal end opening of the elongate inversion support catheter when the end of the tractor tube is pulled into the inversion support catheter, for example, by pulling on the elongate puller coupled to a first end of the tractor tube that is within the inversion support catheter. Thus, as mentioned above, the inversion support catheter must have a sufficient column strength so that it does not buckle or collapse as the tractor tube is pulled into the distal end of the inversion support catheter, even when pulling a hard clot.

The inversion support catheter may be configured so that it does not collapse (e.g., buckle) when 500 g or less of of compressive force is applied (e.g., at least about 700 g, 600 g, 500 g, 400 g, 300 g, etc. of compressive force), particularly when the apparatus is configured for neurovascular applications. For peripheral vascular applications, the elongate inversion support catheter may be selected or configured to withstand at least 1500 g of compressive force (e.g., at least about 2000 g, 1900 g, 1800 g, 1700 g, 1600 g, 1500 g, 1400 g, etc. of compressive force). In addition, the inversion support catheters disclosed and described herein may be configured so that they do not foreshorten substantially when the tractor tube is pulled over the distal end (e.g., do not foreshorten by more than 2%, 3%, 4%, 5%, 6%, 7%, 8%, 9%, 10%, etc.).

It may also be desirable that the inversion support catheters maintain sufficient column strength even when tracked or tracking though highly tortious anatomical regions. FIG. 12 is an example of a tortious neuroanatomical region, showing a drawing of the internal carotid artery, which includes multiple regions (labeled as regions 1, 2, 3, 4 and 5 in FIG. 12). The inversion support catheters disclosed and described herein may have sufficient column strength and may have a structure configured to resist buckling and compression when pulling a tractor tube so that it rolls into the distal end opening of the inversion support catheter (often applying up to 500g of force or more in compression), yet still be sufficiently flexible to track through such tortious regions of the vasculature including regions 1-5 of an internal carotid artery such as the example shown in FIG. 12.

A number of inversion support catheter designs have been examined by the applicants. Many of the inversion support catheters tested, even those having cut-our regions that enhance flexibility, and those with support structures to enhance column strength (e.g., withstanding up to 500 g or more of compression force), do not have both a sufficient column strength to resist buckling/compression and a sufficient flexibility to navigate a region having a tortuosity equivalent to that in a model internal carotid artery, such as the one shown in FIG. 12. Disclosed and described herein are inversion support catheters configured to meet these criteria. In particular, disclosed and described herein are inversion support catheters having a plurality of slots ("slotted inversion support catheters") in which the slots are configured to provide both column strength and flexibility. Slots are typically formed substantially transverse to the long axis of the the catheter, wherein substantially transvers includes e.g., +/- a few degrees off transverse (e.g., +/- 2 degrees, +/- 3 degrees, +/- 4 degrees, +/- 5 degrees +/- 7 degrees, +/- 10 degrees,+/- 12 degrees, etc.). The slots may be cuts through the catheter that extend approximately transverse to the length of the inversion support catheter (at a pitch of between +/- a few degrees, e.g., a pitch of between 0.05 to 0, etc.). The slots may extend, for example, between 60 and 180 degrees (e.g., between 90 and 180 degrees, between 100 and 170 degrees, between 110 and 160 degrees, between 120 and 150 degrees, etc.) around the circumference of the inversion support catheter.

In some of the variations disclosed and described herein, the slots are formed, e.g., by laser cutting, rectangular cut-out regions. The slots may be configured as compressed slots, e.g., by heat-setting the catheter or a portion of the catheter into a longitudinally compressed state. In the compressed state, the slots be configured so that the ends of the slots, which are not typically compressible, have a fixed width, and the region between the ends of the slots is compressed so that the width of this region is less than the width of the ends of the slots. See, e.g., FIGS. 13A-13C. For example, In FIG. 13A a "neutral" slot is shown cut through a catheter, and typically curving around the radius of the catheter. The slot shown in FIG. 13A has end regions 1301, 1305 of the slot that each have a width (w₁) that is approximately the same width as the middle region 1303 between the ends (w₂). 1 inch = 2.54 cm. For example, the width may be between 0.01 and 0.0001 inches (e.g., between 0.005 and 0.0001, between 0.001 and 0.0001, etc.). The neutral slot shown in FIG. 13A may be compressed, e.g., by applying a compressive force on the long axis of the catheter and heat-setting the catheter in the compressed configuration. This may form a compressed slot, also referred to herein as a "closed" slot or "closed cell" slot. In FIG. 13B a closed rectangular slot is shown, having edge regions that have a width (w₁) that is substantially the same as in the neutral position, however the middle region 1303 between the ends 1301, 1305 has a width (w2) that is less than the width at the ends of the slot (e.g., w2 < w₁, e.g., w₂ < 95% w₁, w₂ < 90%w₁, w₂ < 85% w₁, etc.).

The slot may also be opened, as shown in FIG. 13C. In some variations, the slot is opened by applying a tension (e.g., by pulling) force on all or a portion of the elongate length of the catheter into which the slot is formed, and heat-setting the slot in the resulting expanded configuration. In FIG. 13C the middle region 1303 of the slot has been expanded to have a width (w₂) that is greater than the width (w₂) of the end regions 1301, 1305. This may be referred to an an open or open cell configuration for the slot. For example, in an open configuration of a rectangular slot, w₂ > w₁. In some variations, in which w₂ is much larger than w₁ (e.g., w₂ >> w₁, e.g., w₂ is greater than about 2x w₁, w₂ is greater than about 2.5x w₁, w₂ is greater than about 3x w₁, w₂ is greater than about 4x w₁, w₂ is greater than about 5x w₁, etc.) these slots may be referred to as super-open slots.

Any of the inversion support catheters disclosed and described herein may be configured so that the distribution of slots is non-uniform along the length of the catheter. For example, the distal end region of a slotted inversion support catheters (e.g., the distal most region of the catheter between 1-50 mm from the distal end, e.g., between 2-30 mm, between 2-20 mm, between 2-10 mm, etc.) may include open-cell slots (all or some open-cell slots. The portion of the catheter proximal to this distal end may include closed cell or normal slots. In some variations, the catheter may include both open-cell and closed-cell slots arranged around and along the distal end region. The percentage of open-cell slots may be, e.g., between 0. 1%-80% of the total number of slots in the distal end region (e.g., between 0.1% to 70%, between 0.1% to 60%, between 0.1% to 50%, between 0.1% to 40%, between 0.1% to 30%, between 0.1% to 20%, between 0.1% to 10%, between 0.1% to 5%, etc.).

As will be described in greater detail herein, in some variations, the catheter, and particularly the distal end region, may include super open-cell slots. In particular, the percentage of super open-cell slots to other open-cell slots and closed-cell slots may be less than 50% (e.g., less than 40%, less than 30%, less than 20%, less than 10%, less than 5%, less than 1%, less than 0.5%, etc.). As mentioned, other slots may be closed-cell slots and/or may have different dimensions. The region of the inversion support catheter proximal to the distal end region may have a slotted configuration of closed-cell slots.

It is hypothesized that closed-cell slots may help provide column strength to an inversion support catheter, since the adjacent walls of the slot are not able to compress much. However, closed-cell slots are less flexible than slots in the neutral configuration or in the open (or super open) configurations described above. Towards this end, variations of catheters having a proximal region that included primarily or exclusively closed-cell slots with a distal end region (e.g., distal 1-50 mm) that was primarily or exclusively open-cell slots were initially examined.

For example, FIG. 14A illustrates three examples of inversion support catheters, 1401, 1402, 1403. These inversion support catheters have a slotted pattern of cuts (formed by laser cutting). The inversion support catheters were laser cutting a length of NiTi tubing. The entire length of the laser-cut catheter was then compressed and heat set to close the cells formed by the laser cutting. The cut-out regions (e.g., cells) were then compressed so that the separation of the opposite sides of the cut-out region transverse to the length of the catheter had a separation of less than 95% of the original cut-out diameter (e.g., if the original cut-out diameters is 0.0254mm (0.001"), the compressed configuration is 0.00254mm (0.0001")) over at least a portion of the slot (e.g., a middle region of the slot).

Catheters having compressed cut-out regions such as this may show good column strength (e.g., resisting up to 500 g of compression force without bucking), however, they generally were not capable of tracking a tortious model of the vasculature such as shown in FIG. 12, and typically failed to track further than the first (1) or second (2) regions of the model.

To improve tracking, catheters having compressed cut-our regions such as those shown in FIGS. 14A were re-heat set at their distal ends while applying a stretching force. Expanding just the distal end region (e.g., the distal 12mm, distal 15 mm, distal 20 mm, distal 30 mm, distal 40 mm, distal 50 mm, etc.) to have larger-diameter gaps (open-cell slots) was performed on all three of the catheters shown in FIG. 14A. In FIG. 14A, the middle 1402 and right devices 1403 were each first compressed and heat-set in a compressed configuration and then the distal ends were expanded to have a uniform stretching and separation of the gaps in the slotted cells, forming open-celled slots in this distal end region. The slots shown have a middle width between the ends of the slots that is the same or slightly larger than the width of the end regions (e.g., the width of the middle region of the slot is between about 100.5% and 120% of the width of the end regions of the slot). These devices 1402, 1403 performed better than catheters having just closed-cell slots, and were able to track slightly further (e.g., to the second (2) or third (3) regions in the internal carotid model show in FIG. 13).

For example, FIGS. 16A-16F illustrate a slotted inversion support catheter first formed to have uniformly closed-cell slots by heat-setting in a longitudinally compressed configuration, and then heat-setting the distal end region in a stretched (e.g., the distal 2 cm) configuration. The apparatus was then jacketed by lamination of a jacket material over the catheter. As shown in FIGS. 16A-16F, such devices had a column strength that was sufficiently high to avoid significant kinking. Although a slight bend occurred beginning at 100g of compression (FIG. 16B), this bend did not increase significantly even up to 500g of compression (FIG. 16F). FIG. 16G shows a slightly enlarged view of the distal end region of the catheter.

FIGS. 17A-17C illustrate, for comparison, other slotted catheters (e.g., catheters having cut-our regions) that kinked in compression. For example, FIG. 17A (shown enlarged in the inset FIG. 17B) is an example of a first catheter having slots cut therein (neutral slot configuration), showing substantial kinking and compression when 400 g of compression was applied. Similarly, FIG.17C (with an enlarged view of the slots shown in FIG. 17D) shows another example of a catheter having somewhat large, open-cell slots along the length of the catheter; this catheter also showed a great deal of kinking and compression. FIG. 17E is another example of a catheter of FIGS. 16A-16G), repeated here to show a side-by-side comparison.

During the process of stretching the distal end of catheters that had been heat-set in a compressed configuration, some catheters (see, e.g., the catheter 1401 on the left in FIG. 14A, shown in greater detail in FIG. 14B), were inadvertently expanded so that apparent "defects" 1409 were formed in a small minority of some of the cells, forming super open-celled slots. These defects resulted in a subset of slots/cells that were opened to a greater degree than other slots, which were either not opened (remained closed cell, having a maximum width of the middle region that is less than the maximum width of the end regions), were neutral (in which the width of the middle region is approximately the same as the width of the end regions), or were only slightly opened by the stretching process, forming open-cell slots (e.g., sots having a maximum width of the inner region that is between 1x and 1.5x the diameter of the end regions, e.g., between 1x and 2x, between 1x and 2.5x, between 1x and 3x). Surprisingly, catheters having these "defect" regions (super open-cell regions having a maximum width of the middle region that is greater than 1.5x the width of the end regions, e.g., greater than 2x, greater than 2.5x, greater than 3x, etc.) had a greater column strength (e.g., less buckling at an applied, e.g., 500 g, compression force) and better tracking. Such devices were able to track through the model of tortious vasculature, such as the internal carotid model shown in FIG. 12, completely, e.g., to region (5) or beyond.

FIG. 15 illustrates an example of one such device including these introduced "defects" in the distal end region of the catheter. The defects in this example are formed in a subset of cells formed by cutting the slots in the distal end region of the catheter. These defects may be referred to as super open-cell slots (compared to the other closed cell and/or open-cell slots in the distal end region) in which the separation between long and opposite sides of the slot are separated by greater than 1.5x the separation of the majority of the closed cell slots in the distal end region. For example, the maximum separation between the opposite long side of the open cell configuration may be greater than 1.5x, greater than 2x, greater than 2.5x, greater than 3x, greater than 3.5x, greater than 4x, greater than 4.5x, greater than 5x, etc. the maximum separation between the opposite long side of the closed-cell slots at the ends of the rectangular slot. The super open-cell (defect) slots may be distributed within the distal end region and may be present or absent from the proximal region (which may also include slots).

For example, the distal end region of the elongate inversion support catheter may include at least two types of slots cut generally transverse to the length of the elongate inversion support catheter through the wall of the elongate inversion support catheter, based on the size of the gap between opposite walls of the slots, including open-cell slots and super open-cell slots (and/or closed-cell slots or neutral slots). The slots may extend around the circumference of the catheter so that there are between 4 and 1 slots per rotation of the catheter (e.g., between 2 and 3, approximately 2.5, etc.). The opposite walls of the slot may be the walls that are transverse or approximately transverse to the long axis of the catheter. The closed cell slots may have a maximum separation between the opposite walls of c inches (e.g., c may be, for example, 0.0001 inches, 0.0002 inches, 0.0005 inches, 0.001 inches, etc.). The open-cell slots and may have a maximum separation between the opposite walls of *o* inches, where *o* is between 1x and 1.5x the width of the ends of the slot w₁ (e.g., *o* is between 1x and 1.5x w₁, e.g., o is between 1x and 2x w₁, *o* is between 1x and 2.5x w₁, *o* is between 1x and 3x w₁, etc.). The super open-cell slots may have a maximum diameter o that is greater than 1.5x w₁, greater than 2x w₁, greater than 2.5x w₁, greater than 3x w₁, greater than 4x w₁, greater than 5x w₁, greater than 6x w₁, greater than 7x w₁, greater than 8x w₁, greater than 10x w₁, etc. The distribution of super open cell slots to other slots may be uniform or non-uniform, including random. The majority of the slots in the distal end region may be closed and/or open-cell slots, particularly compared to super open-cell slots. For example, there may be between 0.001% and 20% super open-cell slots in the distal end region (e.g., between 0.001% and 10%, between 0.001% and 5%, between 0.01% and 5%, between 0.1% and 5%, etc., between 0.001% and 1%, etc.). For example, there may be between 0.5 and 20 super open-cell slots for every cm length of catheter (e.g., between 1 and 20, between 0.5 and 15, between 0.5 and 10, between 1 and 10, etc.). The super open-cell slots may be oriented at different radial positions around the catheter.

FIG. 14C illustrates an exemplary laser cut pattern for an inversion support catheter such as the ones shown in FIG. 14A. As mentioned above, a length of NiTi tubing may be cut (e.g., laser cut) to form slots, and these slots may be compression heat set along the entire length of the catheter to form closed-cell slots. The catheter may then be re-heat set with the distal end region stretched, to form open-cell slots. In some variations, a small number of these slots may also be formed as super open-cell slots. In FIG. 14C, a variety of pitches are shown (e.g., between 0 and 0.01) for the slots.

FIG. 15 illustrates an example of an inversion support catheter in which the distal 5 mm of the catheter were treated (by stretching and heat setting) to introduce two "defects" (super open-cell regions) 1509. Although the majority of the slots in the distal end remained simply open-cell slots, these super open-cell slots (which have a maximum width of greater than 1.5x the width of the ends of the rectangular/oval slot) greatly enhanced the flexibility and tracking of the catheter; the exemplary catheter in FIG. 15 also had a column strength sufficient so that little or no kinking resulted even when applying up to 500 g of compressive force, as may be applied when rolling a tractor tube into the catheter to capture a hard and/or large clot.

In any of the apparatuses disclosed and described herein, distal end region (e.g., the distal 1-50 mm, distal 2-10 mm, etc.) may have open-celled slots (and/or super open-cell slots) compared the more proximal regions, which may be formed of closed cell slots. For example, FIGS. 18A-18E illustrate examples of inversion support catheters having a combination of open cell and closed cell cut-out regions. In FIG. 18A, the distal end region includes open cells, which may be configured as described above. In FIG. 18A, the majority or all of the distal end region include open-celled slots, compared to the proximal region which are closed-cell slots. In FIG. 18B, a percentage of the cut-out slots are open-celled, but not all (e.g., between 0.1% and 80%, etc.) of the slots are open-cell slots. In FIG. 18C, the distal end region includes slots that are tapered in the middle (e.g., "hourglass" slots) so that each slot includes open-celled and closed-cell regions; the proximal region is shown as all closed-cell slots. Such bi-lobed slots (also referred to as hourglass, barbell and/or dog bone slots) may also have improved tracking and compression strength, comparable to the device having super open-cell slots discussed above; these will be described in greater detail below.

In FIG. 18D the distal end region includes an open coil configuration. In FIG. 18E, the distal coil region is referred to as "closed coil" as the adjacent coils are spaced (over at least a portion of their length) very close together. Although FIGS. 18A-18E show catheters having a stark transition from the proximal and distal end regions, in some variations the transition may be more gradual, so that the device transitions gradually from close cell (proximal) to open-cell (distal) cut-out regions.

FIGS. 19A and 19B illustrate open and closed cell configurations of cut-out regions that may be formed by heat-setting the catheter in compression. In FIG. 19A, a rectangular slot is cut from the catheter as described above in an open configuration, having a separation between the opposite long walls of the slot that is z₁ wide (shown on left) in the closed configuration (under compression) the slot may be heat-set to have a diameter over the middle (x₂) region that is z₂ wide. The edge region of the slot (e.g., regions x₁ and x₃) may be nearly incompressible near their edges and may therefore remain separated. As described in FIG. 19C, the design of FIG. 19A may have dimensions in the closed cell configuration so that x₂ is between about 5% of x₁ and 95% of x₁. For example, in the compressed, close-cell configuration, the compressed region x2 may be the middle 1% to 90% of the total length (x) of the slot. Similarly, the separation z₂ between in the long walls of the slot in the compressed configuration may be less than or equal to 95% of the original separation, z₁. 1 inch = 2.54 cm. For example, if the original separation (z₁) is 0.001 inches, the compressed separation (z₂) may be approximately 0.0001 inches. Generally, the open cell separation may be between, for example, 0.0005 inches and 0.010 inches.

The centrally-tapered slots (hourglass configuration) shown in FIG. 19B in the open-cell and closed-cell configurations may include larger width regions (y₁) one either side of the slot, separated by a narrow region z₁. The lengths of the wider regions (x₁, x₃) and the intervening narrower region (x₂) may add up to the total length of the slot, x. In the closed cell configuration, the separation of the wider regions (y₁) may remain approximately the same, while the middle region, z₂, may be much smaller than the original, open-cell width, z₁. As exemplified in FIG. 19C, the configuration of FIG. 19B, may have a closed cell configuration in which the closed cell width z₂ is less than or equal to 90% of the original open-cell width z₁. The narrower regions is typically centered (but may be offset) within the slot, and may include the middle 5%-95% of the slot. For example, an hourglass-cut slot such as the one shown in FIG. 19B may have an open-cell maximum width of between 0.0005 inches and 0.010 inches at the wide regions on either end and a narrower central region that is between about 10% and 50% of the width of the wider ends (y₁).

FIGS. 20A-20C illustrate alternative configuration of slots that may be cut into the catheter having a centrally-tapered (e.g., hourglass- or barbell- shape) configuration. These configurations typically include an open cell region even in the compressed configuration.

FIGS. 21A-21C illustrate additional slotted catheter designs having regions of closed and open sections that may be formed by cutting or patterning, without requiring heat setting. Alternatively, or additionally, heat setting may be used. In FIG. 21A, the cut pattern 2101 is shown, with slots arranged in an overlapping pattern (a brick stacked pattern). FIG. 21B shows a side view, showing the pattern of alternating slots arranged adjacently along the length of the catheter so that there are columns of high-compression strength in the noon 2103, 3 o'clock 2103', 6 o'clock 2103", and 9 o'clock 2103'" positions radially around the circumference of the slotted catheter (or the slotted portion of the catheter). FIG. 21C shows a side perspective view of the slotted catheter. Other overlapping patterns may be used, including patterns in which high-support regions 2103, 2103', 2103", 2103'" line up along the long length of the catheter in more or fewer than the 4 radial locations shown in FIG. 21A-21C (e.g., in the noon, 4 o'clock, 8 o'clock position, etc.). In some variations the overlapping pattern creates a spiral or helical pattern of high-support regions along the length of the catheter. As mentioned, this pattern may also be compressed and heat-set in a closed-cell configuration. Further cells can be open-cell or closed-cell in different regions along the length of the catheter.

Another example of a slotted catheter design is shown in FIG. 22A-22C. FIG. 22A shows the cut pattern 2201 comprising a narrow spiral cut pattern. The open-cell spiral design pattern may be shaped (e.g., by applying compression and/or pulling longitudinal force and shape-setting) to be open-cell or closed-cell along the entire length of along portions of the length of the catheter. In FIGS. 22A-22C, the spiral pattern includes aligned overlapping/adjacent regions so that there are five columns of higher-support regions down the elongate length of the catheter. FIG. 22B shows a side view, and FIG. 22C shows a side perspective view.

FIGS. 23A-23C illustrate another variation of a slotted catheter pattern design. In this example, the slotted pattern is a dog bone pattern, as shown in FIG. 23A. Each cut-out slot has a distal end region with a large width between the opposite sides, and a narrow connection region, with a much smaller width between the opposite sides. The narrow-width regions may be aligned in adjacent slots along the length of the catheter, as shown in FIGS. 23B and 23C. In this example, this alignment of the narrow regions in the middle of the slot (similar to the "closed-cell rectangular slots described above) result in four columns of high-compression strength in the noon 2303, three o'clock 2303', six o'clock 2303" and nine o'clock 2303'" positions radially around the circumference of the slotted catheter (or the slotted portion of the catheter). In this example, each cell has preferred close contact region. As mentioned above, the cells (or regions of the cells along the length of the catheter) can be compressed or stretched and heat-set in an open-cell or closed-cell configuration.

In addition to mixing regions of closed-cell and open-cell slots along the length of the catheter, any of these catheters may also include a mixture of the different patterns disclosed and described herein. For example, FIG. 24A shows an example in which the spiral pattern 2403 of FIGS. 22A-22C is shown adjacent to a region of the brick pattern 2405 of FIG. 21A-21C, which is also adjacent to another region of the spiral pattern. Similarly, FIG. 24B shows alternating spiral patterns 2403 and barbell patterns 2407 such as that shown in FIG. 23A-23C. FIG. 24C shows alternating patterns of spiral 2403, brick 2405, spiral 2403, barbell/dog bone 2407, etc. In any of these catheters, including those of FIGS. 21A-24C, regions along the length may be compressed and/or stretched to form open-cell and closed-cell slots as well.

FIGS. 25A-25C illustrate a continuous spiral-cut slotted pattern 2501 in which a slot includes plurality of narrowed regions (bumps, detents, necks, etc.) in the slot and aligned along the longitudinal axis of the catheter to form lines 2503', 2503", 2503'" extending in the long, longitudinal axis of the catheter. As with the embodiments described above, these lines may increase the column strength while the larger-width cut-out regions between them may enhance flexibility. In FIG. 25A, the cut pattern 2501 is shown. As mentioned, the cut pattern may be continuous, and may spiral around and through the catheter (compare to the discrete patterns of FIGS. 21A-24C, which also spiral around the catheter at a very low pitch). FIG. 25B shows a side view, including the three lines of aligned narrowed regions. Although three lines are shown, a greater or lower number of narrowed regions may be included (e.g., one or more, two or more, three or more, four or more, 2 to 60, 2 to 50, 2 to 40, 2 to 30, 2 to 25, 2 to 20, 2 to 19, etc.), and spaced so that they align to form longitudinally-extending lines. The lines may be parallel with the longitudinal axis of the catheter. In some variations, the lines may spiral around the catheter with a pitch of less than 15 degrees (e.g., less than 14 degrees, less than 12 degrees, less than 10 degrees, less than 8 degrees, less than 6 degrees, less than 5 degrees, etc.). As with the variations shown in any of FIGS. 14A to 24C, the variation shown in FIGS. 25A-25C may be used in order to achieve a high degree of flexibility and column stiffness when pulling a tractor tube into the catheter, as described above, particularly when this pattern is included on the distal end of the catheter (e.g., the distal 30 cm, the distal 25 cm, the distal 20 cm, the distal 15 cm, the distal 20 cm, etc.).

FIG. 26 illustrates another example of an apparatus including an elongate inversion support catheter comprising a plurality of slots that are arranged approximately transversely to a long axis of the elongate slotted inversion support catheter. In this example, the apparatus includes a first region of the slotted inversion support catheter that has a "crushed spiral" arrangement in which the spiral shape for the slots is formed by cutting (e.g., laser cutting) slots around the circumference, applying a compressive force along the length and heat setting it to hold the compressed format. Adjacent to this first (distal region) is a second region forming a stretch of 'crushed brick' configuration in child wherein the slots have an open diameter of about 0.001 inches or more and wherein there are between 2 and 4 slots per circumferential turn about the long axis, and the region has been compressed (compressively loaded) then heat treated to retain the compressed form. Finally, in FIG. 26, a third slotted region is proximal to the second one is shown. In this third embodiment the third one is an "open brick" configuration in which the slots cut transversely to the long axis of the elongate slotted inversion support catheter, have an open diameter of about 0.001 inches or more and wherein there are between 2 and 4 slots per circumferential turn about the long axis. Any combination of slotted regions (spirals, bricks, open, closed) may be used, in any order of particular interest. Also shown in FIG. 26 is an example of a ring of metal (e.g. shape memory alloy, such as TiNi, or stainless steel, etc. In FIG. 26, the catheter has an ID of 1.2192mm (0.048") and an outer diameter (OD) of about 1.5748mm (0.062"). The catheter is formed of two separate laser-cut hypotubes that have been joined together as shown in FIG. 27 (showing a stainless-steel member interlocking with a nitinol at dovetail joints; a coating or sleeve (e.g., a lamination such as with Tecoflex 80A and Pebax) may be used to secure them together without substantially modifying the flexibility of the catheter. The length of the nitinol catheter cut to form the distal end of the catheter may be approximately 65cm (e.g., between 40 cm and 100 cm, between 45cm and 90cm, between 55 cm and 85, etc.). The length of the stainless steel (SS) catheter cut to form the distal end of the catheter may be approximately 100 cm (e.g., between 50 cm and 150 cm, between 75 cm and 120 cm, between 95 cm and 105 cm, etc.). The regions of the catheter be treated to have a yield compression strength of greater than ~1000grm.

FIG. 28 illustrates an example of a catheter, e.g., an elongate inversion support catheter, such as the one shown in FIG. 26, comprising a plurality of slots arranged approximately transversely to a long axis of the elongate slotted inversion support catheter, wherein the slots have an open diameter of about 0.001 inches or less and wherein there are between 2 and 4 slots per circumferential turn about the long axis. FIG. 28, the catheter is shown making a tight turn of 2.8 mm diameter, showing good tracking which may be reflected in the body. Similarly, FIGS. 29A and 29B illustrate an example of a comparison between bending of a traditional support catheter and an elongate inversion support catheter (comprising a plurality of slots arranged approximately transversely to a long axis) as disclosed and described herein. In FIG. 29A, the traditional catheter shows buckling and ultimately fails. In contrast, in FIG. 29B the same applied force (e.g., compressive force) does not cause the device (the elongate inversion support catheter. Thus, the improved elongated inversion support catheter handles high compressive loads without snaking/accordioning/buckling when ingesting hard clot.

The exemplary methods and apparatuses disclosed and described herein may be used with all or some portions of the mechanical clot removal devices shown in each of: U.S. 2017086864, filed on 10/11/2016 ("Mechanical Thrombectomy Apparatuses and Methods"); U.S. 017303948, filed on 4/25/2017 ("Anti-Jamming and Macerating Thrombectomy Apparatuses and methods"), U.S. 2017303942, filed on 4/25/2017 ("Pre-loaded inverting tractor Thrombectomy Apparatuses and methods"); U.S. 2017303939, filed on 4/25/2017 ("Methods for Advancing Inverting Mechanical Thrombectomy Apparatuses In The Vasculature"); U.S. 2017303947, filed on 4/25/2017 ("Clot-Engulfing Mechanical Thrombectomy Apparatuses and methods of use"); and U.S. 2017348014, filed on 6/1/2017 ("Inverting Thrombectomy Apparatuses And Methods").

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the various inventions disclosed and described herein. For example, as used herein, the singular forms "a", "an" and "the" are intended to include the plural forms as well, unless the context clearly indicates otherwise. It will be further understood that the term "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed herein could be termed a second feature/element, and similarly, a second feature/element discussed herein could be termed a first feature/element without departing from the teachings of the disclosed and described embodiments.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and exemplary methods disclosed and described herein should be understood to be inclusive, but all or a sub-set of the components and/or steps may alternatively be exclusive and may be expressed as "consisting of' or alternatively "consisting essentially of' the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the disclosed inventions, which are defined by the claims. Optional features of various device and system embodiments may be included in some embodiments and not in others. Therefore, the foregoing description is provided primarily for exemplary purposes and should not be interpreted to limit the scope of the inventions as they are set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, as many are, in fact, disclosed. Thus, although specific embodiments have been illustrated and disclosed and described herein, any arrangement calculated to achieve the same purpose may be substituted for the specific embodiments shown. This disclosure is intended to cover any and all adaptations or variations of the various embodiments disclosed and described herein, as well as further embodiments not specifically disclosed and/or described herein, will be apparent to those of skill in the art upon reviewing the above description in conjunction with the accompanying drawings. The invention is defined by the following claims.

## Claims

1. A mechanical thrombectomy apparatus for removing a clot (1127) from a vessel, the apparatus comprising:
an elongate inversion support catheter (1005; 1105) having a distal end and a distal end opening;
an elongate puller (1007; 1027; 1047; 1107; 1107') extending within the elongate inversion support catheter (1005; 1105); and
a tractor tube (1003; 1103) extending over an outer surface of the elongate inversion support catheter (1005; 1105), inverting into the distal end opening of the elongate inversion support catheter (1005; 1105), and attached to the elongate puller (1007; 1027; 1047; 1107; 1107') at a first end within the elongate inversion support catheter (1005; 1105),
**characterized in that**
the elongate puller (1007; 1027; 1047; 1107; 1107') is configured as a pull wire and comprises, at a distal end of the elongate puller, a cannulated nose (1009, 1109) configured to allow passage of a guidewire therethrough.

2. The apparatus of claim 1, wherein the cannulated nose (1009; 1109) is a constant stiffness structure.

3. The apparatus of one of claims 1 and 2, wherein the tractor tube (1003, 1103) is coupled proximal to the distal end of the elongate puller (1007; 1027; 1047; 1107; 1107').

4. The apparatus of claim 1, wherein the distal end (1109) of the puller (1107; 1107') is tapered.

5. The apparatus of claim 4, wherein the distal end (1109) of the puller (1107; 1107') includes a plurality of projections (1115; 1125; 1137) extending from the distal end (1109).

6. The apparatus of claim 5, wherein the projections (1115) are configured as cups that have proximal-facing edges.

7. The apparatus of one of claims 5 and 6, wherein the projections (1115; 1125; 1137) extend all or partially around an entire tip of the distal end (1109) of the elongate puller (1007; 1027; 1047; 1107; 1107').

8. The apparatus of any one of claims 5 to 7, wherein the more distal projections (1115; 1125; 1137) have smaller diameters than more proximal projections (1115; 1125; 1137).

9. The apparatus of claim 5, wherein the projections (1125) are configured as fibers or fingers extending from an outer surface of the distal end (1109) of the puller (1107').

10. The apparatus of claim 9, wherein the projections (1125) are in one or more sets of projections directed proximally.

11. The apparatus of claim 5, wherein the projections (1125) are constructed from an open-end braid feature.

12. The apparatus of claim 1, wherein the elongate puller (1107; 1107') comprises one or more helical projections (1137) extending proud of the outer surface of the elongate puller (1107; 1107') and extending around the elongate puller (1107; 1107').

13. The apparatus of claim 12, wherein the one or more helical projections (1137) are formed of a wire wrapped around the distal end of the puller (1107).

14. The apparatus of any one of claims 1 to 13, wherein the tractor tube (1003, 1103) is knitted.

## Patentansprüche

1. Mechanische Thrombektomievorrichtung zum Entfernen eines Gerinnsels (1127) aus einem Gefäß, wobei die Vorrichtung umfasst:
einen länglichen Inversions-Stützkatheter (1005; 1105) mit einem distalen Ende und einer Öffnung an dem distalen Ende;
eine längliche Zugeinrichtung (1007; 1027; 1047; 1107; 1107'), die sich innerhalb des länglichen Inversions-Stützkatheters (1005; 1105) erstreckt; und
einen Schleppschlauch (1003; 1103), der sich über eine Außenfläche des länglichen Inversions-Stützkatheters (1005; 1105) erstreckt, in die Öffnung am distalen Ende des länglichen Inversions-Stützkatheters (1005; 1105) hinein umbiegt und an einem erste Ende an der länglichen Zugeinrichtung (1007; 1027; 1047; 1107; 1107') innerhalb des länglichen Inversionsstützkatheters (1005; 1105) befestigt ist, **dadurch gekennzeichnet, dass**
die längliche Zugeinrichtung (1007; 1027; 1047; 1107; 1107') als Zugdraht gestaltet ist und an einem distalen Ende der länglichen Zugeinrichtung eine kanülierte Nase (1009, 1109) umfasst, die dafür ausgelegt ist, den Durchgang eines Führungsdrahtes durch sie hindurch zu ermöglichen.

2. Vorrichtung nach Anspruch 1, wobei die kanülierte Nase (1009; 1109) eine Struktur mit konstanter Steifigkeit ist.

3. Vorrichtung nach einem der Ansprüche 1 und 2, wobei der Schleppschlauch (1003, 1103) proximal mit dem distalen Ende der länglichen Zugeinrichtung (1007; 1027; 1047; 1107; 1107') gekoppelt ist.

4. Vorrichtung nach Anspruch 1, wobei sich das distale Ende (1109) der Zugeinrichtung (1107; 1107') verjüngt.

5. Vorrichtung nach Anspruch 4, wobei das distale Ende (1109) der Zugeinrichtung (1107; 1107') eine Mehrzahl von Vorsprüngen (1115; 1125; 1137) aufweist, die sich von dem distalen Ende (1109) aus erstrecken.

6. Vorrichtung nach Anspruch 5, wobei die Vorsprünge (1115) als Kelche gestaltet sind, die proximal zugewandte Ränder aufweisen.

7. Vorrichtung nach einem der Ansprüche 5 und 6, wobei sich die Vorsprünge (1115; 1125; 1137) ganz oder zum Teil um eine gesamte Spitze des distalen Endes (1109) der länglichen Zugeinrichtung (1007; 1027; 1047; 1107; 1107') erstrecken.

8. Vorrichtung nach einem der Ansprüche 5 bis 7, wobei die mehr distalen Vorsprünge (1115; 1125; 1137) kleinere Durchmesser aufweisen als mehr proximalen Vorsprünge (1115; 1125; 1137).

9. Vorrichtung nach Anspruch 5, wobei die Vorsprünge 1125) als Fasern oder Finger gestaltet sind, die sich von einer Außenfläche des distalen Endes (1109) der Zugeinrichtung (1107') aus erstrecken.

10. Vorrichtung nach Anspruch 9, wobei die Vorsprünge (1125) in einer oder mehreren Vorsprungsgruppen liegen, die proximal ausgerichtet sind.

11. Vorrichtung nach Anspruch 5, wobei die Vorsprünge (1125) aus einem offenendigen geflochtenen Gebilde konstruiert sind.

12. Vorrichtung nach Anspruch 1, wobei die längliche Zugeinrichtung (1107; 1107') einen oder mehrere spiralförmige Vorsprünge (1137) aufweist, die über die Außenfläche der länglichen Zugeinrichtung (1107; 1107') vorstehen und sich um die länglichen Zugeinrichtung (1107; 1107') herum erstrecken.

13. Vorrichtung nach Anspruch 12, wobei der eine oder die mehreren spiralförmigen Vorsprünge (1137) aus einem Draht gebildet sind, der um das distale Ende der Zugeinrichtung (1107) gewickelt ist.

14. Vorrichtung nach einem der Ansprüche 1 bis 13, wobei der Schleppschlauch (1003, 1103) gestrickt ist.

## Revendications

1. Appareil mécanique de thrombectomie destiné à éliminer un caillot (1127) à partir d'un vaisseau, l'appareil comprenant:
un cathéter de support allongé (1005 ; 1105) du type à inversion qui possède une extrémité distale et une ouverture à l'extrémité distale ;
un élément de traction allongé (1007 ; 1027 ; 1047 ; 1107 ; 1107') qui s'étend au sein du cathéter de support allongé (1005 ; 1105) du type à inversion ; et
un tube de traction (1003; 1103) qui s'étend par-dessus une surface externe du cathéter de support allongé (1005 ; 1105) du type à inversion, qui s'inverse dans l'ouverture à l'extrémité distale du cathéter de support allongé (1005 ; 1105) du type à inversion, et qui est fixé à l'élément de traction allongé (1007; 1027; 1047; 1107; 1107') à une première extrémité au sein du cathéter de support allongé (1005; 1105) du type à inversion ;
**caractérisé en ce que**
l'élément de traction allongé (1007 ; 1027; 1047; 1107; 1107') est configuré sous la forme d'un câble de traction et comprend, à une extrémité distale de l'élément de traction allongé, un nez cannelé (1009, 1109) qui est configuré pour qu'un fil de guidage puisse le traverser.

2. Appareil selon la revendication 1, dans lequel le nez cannelé (1009, 1109) possède une structure du type à rigidité constante.

3. Appareil selon l'une quelconque des revendications 1 et 2, dans lequel le tube de traction (1003 ; 1103) est couplé, en position proximale, à l'extrémité distale de l'élément de traction allongé (1007; 1027; 1047; 1107; 1107').

4. Appareil selon la revendication 1, dans lequel l'extrémité distale (1109) de l'élément de traction allongé (1107 ; 1107') est de forme conique.

5. Appareil selon la revendication 4, dans lequel l'extrémité distale (1109) de l'élément de traction allongé (1107 ; 1107') englobe un certain nombre de saillies (1115 ; 1125 ; 1137) qui s'étendent à partir de l'extrémité distale (1109).

6. Appareil selon la revendication 5, dans lequel les saillies (1115) sont configurées sous la forme de cupules qui possèdent des bords qui sont orientés dans la direction proximale.

7. Appareil selon l'une quelconque des revendications 5 et 6, dans lequel les saillies (1115 ; 1125 ; 1137) s'étendent totalement ou en partie autour d'une pointe entière de l'extrémité distale (1109) de l'élément de traction allongé (1007 ; 1027 ; 1047 ; 1107 ; 1107').

8. Appareil selon l'une quelconque des revendications 5 à 7, dans lequel les saillies plus distales (1115; 1125; 1137) possèdent des diamètres qui sont inférieurs à ceux des saillies plus proximales (1115 ; 1125 ; 1137).

9. Appareil selon la revendication 5, dans lequel les saillies (1125) sont configurées sous la forme de fibres ou de doigts qui s'étendent à partir d'une surface externe de l'extrémité distale (1109) de l'élément de traction allongé (1007').

10. Appareil selon la revendication 9, dans lequel les saillies (1125) sont disposées en formant un ou plusieurs groupes de saillies qui sont orientées dans la direction proximale.

11. Appareil selon la revendication 5, dans lequel les saillies (1125) sont conçues à partir d'un élément réalisé sous la forme d'une tresse du type à extrémité ouverte.

12. Appareil selon la revendication 1, dans lequel l'élément de traction allongé (1107 ; 1107') comprend une ou plusieurs saillies (1137) de configuration hélicoïdale qui s'étendent de manière protubérante de la surface de l'élément de traction allongé (1107; 1107') et qui s'étendent autour de l'élément de traction allongé (1107 ; 1107').

13. Appareil selon la revendication 12, dans lequel lesdites une ou plusieurs projections de configuration hélicoïdale (1137) sont conçues sous la forme d'un fil métallique qui vient s'enrouler autour de l'extrémité distale de l'élément de traction (1107).

14. Appareil selon l'une quelconque des revendications 1 à 13, dans lequel tube de traction (1003 ; 1103) est tricoté.
